# EUROPEAN PATENT SPECIFICATION

(11) **EP 2 806 754 B1**
(45) Date of publication and mention of the grant of the patent: **24.10.2018**
(21) Application number: 13700929.6
(22) Date of filing: 23.01.2013
(51) Int. Cl.: C12N 9/02, C12N 9/10, C12N 9/90, A23L 2/60, C12P 5/00, C07H 15/256, C12P 19/56, C12N 15/52, A23K 20/121, A23K 20/163, A23K 20/10, A23L 27/30, C12N 9/88

(54) **DITERPENE PRODUCTION**
HERSTELLUNG VON DITERPEN
PRODUCTION DE DITERPÈNE

(30) Priority: 23.01.2012 US 201261589683 P; 16.11.2012 US 201261727591 P
(43) Date of publication of application: 03.12.2014
(73) Proprietor: DSM IP Assets B.V., 6411 TE Heerlen (NL)
(72) Inventor: KUMAR, Manoj, Parsippany, New Jersey 07054 US (US); VAN DEN BERG, Marco Alexander, 6100 AA Echt (NL); BOER, Viktor Marius, NL-6100 AC Echt (NL); LAWRENCE, Adam G., MA 02142 Lexington Massachussets (US); LEHMANN, Martin, CH-4303 Kaiseraugst (CH); MEIJRINK, Bernard, NL-6100 AC Echt (NL); SAGT, Cornelis Maria Jacobus, NL-6100 AC Echt (NL); TRUEHART, Joshua, NL-6100 AA Echt (NL); WU, Liang, NL-6100 AC Echt (NL); ZWARTJENS, Priscilla, NL-1600 AC Echt (NL)
(74) Representative: DSM Intellectual Property
(86) International application number: PCT/EP2013/051262
(87) International publication number: WO 2013/110673

(56) References cited:
- EP-A2- 1 897 951
- WO-A1-2010/104763
- WO-A1-2011/153378
- WO-A1-2012/075030
- WO-A2-2009/108680
- US-A1- 2004 072 323
- US-A1- 2010 112 156
- US-A1- 2010 297 722
- PARAYIL KUMARAN AJIKUMAR ET AL: "Terpenoids: opportunities for biosynthesis of natural product drugs using engineered microorganisms", MOLECULAR PHARMACEUTICS, AMERICAN CHEMICAL SOCIETY, US, vol. 5, no. 2, 1 March 2008 (2008-03-01), pages 167-190, XP002617175, ISSN: 1543-8384, DOI: 10.1021/MP700151B [retrieved on 2008-03-21]
- KIRBY JAMES ET AL: "Biosynthesis of plant isoprenoids: perspectives for microbial engineering", ANNUAL REVIEW OF PLANT BIOLOGY,, vol. 60, 1 January 2009 (2009-01-01), pages 335-355, XP009143317, DOI: 10.1146/ANNUREV.ARPLANT.043008.091955
- BRANDLE ET AL: "Steviol glycoside biosynthesis", PHYTOCHEMISTRY, PERGAMON PRESS, GB, vol. 68, no. 14, 1 July 2007 (2007-07-01), pages 1855-1863, XP022145443, ISSN: 0031-9422, DOI: 10.1016/J.PHYTOCHEM.2007.02.010
- TANIA V HUMPHREY ET AL: "Spatial Organisation of Four Enzymes from Stevia rebaudiana that are Involved in Steviol Glycoside Synthesis", PLANT MOLECULAR BIOLOGY, KLUWER ACADEMIC PUBLISHERS, DORDRECHT, NL, vol. 61, no. 1-2, 1 May 2006 (2006-05-01), pages 47-62, XP019405427, ISSN: 1573-5028, DOI: 10.1007/S11103-005-5966-9
- HITESH KUMAR ET AL: "A comprehensive analysis of fifteen genes of steviol glycosides biosynthesis pathway in(Bertoni)", GENE, ELSEVIER, AMSTERDAM, NL, vol. 492, no. 1, 4 October 2011 (2011-10-04), pages 276-284, XP028345135, ISSN: 0378-1119, DOI: 10.1016/J.GENE.2011.10.015 [retrieved on 2011-10-20]
- NORIHIKO MISAWA: "Pathway engineering for functional isoprenoids", CURRENT OPINION IN BIOTECHNOLOGY, vol. 22, no. 5, 1 October 2011 (2011-10-01), pages 627-633, XP55012413, ISSN: 0958-1669, DOI: 10.1016/j.copbio.2011.01.002
- REILING K KINKEAD ET AL: "Mono and diterpene production in Escherichia coli", BIOTECHNOLOGY AND BIOENGINEERING, WILEY & SONS, HOBOKEN, NJ, US, vol. 87, no. 2, 20 July 2004 (2004-07-20), pages 200-212, XP009143370, ISSN: 0006-3592, DOI: 10.1002/BIT.20128 [retrieved on 2004-06-18]

## Description

### Field

The present disclosure relates to a recombinant microorganism capable of producing a diterpene and/or a glycosylated diterpene and to a process for the production of a diterpene and/or a glycosylated diterpene by use of such a cell. The disclosure further relates to a fermentation broth comprising a diterpene and/or glycosylated diterpene obtainable by such a process.

### Background

The worldwide demand for high potency sweeteners is increasing and, with blending of different artificial sweeteners, becoming a standard practice. However, the demand for alternatives is expected to increase. The leaves of the perennial herb, *Stevia rebaudiana* Bert., accumulate quantities of intensely sweet compounds known as steviol glycosides. Whilst the biological function of these compounds is unclear, they have commercial significance as alternative high potency sweeteners, with the added advantage that *Stevia* sweeteners are natural plant products.

These sweet steviol glycosides have functional and sensory properties that appear to be superior to those of many high potency sweeteners. In addition, studies suggest that stevioside can reduce blood glucose levels in Type II diabetics and can reduce blood pressure in mildly hypertensive patients.

Steviol glycosides accumulate in Stevia leaves where they may comprise from 10 to 20% of the leaf dry weight. Stevioside and rebaudioside A are both heat and pH stable and suitable for use in carbonated beverages and many other foods. Stevioside is between 110 and 270 times sweeter than sucrose, rebaudioside A between 150 and 320 times sweeter than sucrose. In addition, rebaudioside D is also a high-potency diterpene glycoside sweetener which accumulates in Stevia leaves. It may be about 200 times sweeter than sucrose

Currently, steviol glycosides are extracted from the *Stevia* plant. In *Stevia,* (-)-kaurenoic acid, an intermediate in gibberellic acid (GA) biosynthesis, is converted into the tetracyclic dipterepene steviol, which then proceeds through a multi-step glucosylation pathway to form the various steviol glycosides. However, yields may be variable and affected by agriculture and environmental conditions. Also, Stevia cultivation requires substantial land area, a long time prior to harvest, intensive labour and additional costs for the extraction and purification of the glycosides.

New, more standardized, clean single composition, no after-taste, sources of glycosides are required to meet growing commercial demand for high potency, natural sweeteners.

### Summary

In Stevia, steviol is synthesized from GGPP, which is formed by the deoxyxylulose 5-phosphate pathway. The activity of two diterpene cyclases (-)-copalyl diphosphate synthase (CPS) and (-)-kaurene synthase (KS) results in the formation of (-)-Kaurene which is then oxidized in a three step reaction by (-)-kaurene oxidase (KO) to form (-)-kaurenoic acid.

In Stevia leaves, (-)-kaurenoic acid is then hydroxylated, by ent-kaurenoic acid 13-hydroxylase (KAH) to form steviol. Steviol is then glucosylated by a series of UDP-glucosyltransferases (UGTs).

This disclosure relates to a microorganism capable of producing a diterpene, such as steviol, or a glycosylated diterpene (i.e. a diterpene glycoside), such as steviolmonoside, steviolbioside, stevioside, rebaudioside A, rebaudioside B, rebaudioside C, rebaudioside D, rebaudioside E, rebaudioside F, rubusoside or dulcoside A.

According to the disclosure, there is thus provided a recombinant microorganism comprising one or more nucleotide sequence(s) encoding:
a polypeptide having ent-copalyl pyrophosphate synthase (CPS) activity;
a polypeptide having ent-Kaurene synthase (KS) activity;
a polypeptide having ent-Kaurene oxidase (KO) activity; and
a polypeptide having kaurenoic acid 13-hydroxylase (KAH) activity,
whereby expression of the nucleotide sequence(s) confer(s) on the microorganism the ability to produce at least steviol.

In particular, the invention provides a recombinant microorganism which is a *Yarrowia,* such as *Yarrowia lipolytica,* comprising one or more nucleotide sequence(s) encoding:
a polypeptide having ent-copalyl pyrophosphate synthase activity;
a polypeptide having ent-Kaurene synthase activity;
a polypeptide having ent-Kaurene oxidase activity;
a polypeptide having kaurenoic acid 13-hydroxylase activity,
a polypeptide having NADPH-cytochrome p450 reductase activity; and
a polypeptide having UDP-glucosyltransferase activity,
wherein a nucleotide sequence encoding a polypeptide having ent-Kaurene oxidase activity encodes a polypeptide comprising an amino acid sequence that has at least 20% sequence identity with the amino acid sequence of SEQ ID NO: 86; and
wherein a nucleotide sequence encoding a polypeptide having kaurenoic acid 13-hydroxylase activity encodes a polypeptide comprising an amino acid sequence that has at least 20% sequence identity with the amino acid sequence of SEQ ID NO: 34,
whereby expression of the nucleotide sequence(s) confer(s) on the microorganism the ability to produce at least one of steviolmonoside, steviolbioside, stevioside or rebaudioside A, rebaudioside B, rebaudioside C, rebaudioside D, rebaudioside E, rebaudioside F, rubusoside, dulcoside A.

The invention also provides a recombinant microorganism of the invention, wherein the microorganism comprises one or more nucleotide sequence(s) encoding one or more polypeptides having UDP-glucosyltransferase activity (UGT),
whereby expression of the nucleotide sequence confers on the microorganism the ability to produce at least one of steviolmonoside, steviolbioside, stevioside, rebaudioside A, rebaudioside B, rebaudioside C, rebaudioside D, rebaudioside E, rebaudioside F, rubusoside or dulcoside A.

The disclosure also provides:
- a process for the preparation of a diterpene or glycosylated diterpene which comprises fermenting a recombinant microorganism of the disclosure in a suitable fermentation medium, and optionally recovering the diterpene or glycosylated diterpene;
- a process for the preparation of a diterpene or glycosylated diterpene which process comprises fermenting a recombinant microorganism capable of producing a diterpene or glycosylate diterpene in a suitable fermentation medium at a temperature of about 29°C or less, and optionally recovering the diterpene or glycosylated diterpene;
- a fermentation broth comprising a diterpene or glycosylated diterpene obtainable by the process of the disclosure;
- a diterpene or glycosylated diterpene obtained by a process according to the disclosure or obtainable from a fermentation broth according to the disclosure;
- a diterpene or glycosylated diterpene according to the disclosure which is rebaudioside A or rebaudioside D; and
- a foodstuff, feed or beverage which comprises a diterpene or glycosylated according to the disclosure.

Also provided by the invention is a method for converting a first glycosylated diterpene into a second glycosylated diterpene, which method comprises:
contacting said first glycosylated diterpene with a microorganism according to the invention,
thereby to convert the first glycosylated diterpene into the second glycosylated diterpene.

### Brief description of the drawings

Figure 1 sets out a schematic representation of the plasmid pUG7-EcoRV.
Figure 2 sets out a schematic representation of the method by which the ERG20, tHMG1 and BTS1 over-expression cassettes are designed (A) and integrated (B) into the yeast genome. (C) shows the final situation after removal of the KANMX marker by the Cre recombinase.
Figure 3 sets out a schematic representation of the ERG9 knock down construct. This consists of a 500 bp long 3' part of *ERG9,* 98 bp of the *TRP1* promoter, the *TRP1* open reading frame and terminator, followed by a 400 bp long downstream sequence of *ERG9.* Due to introduction of a Xbal site at the end of the *ERG9* open reading frame the last amino acid changes into Ser and the stop codon into Arg. A new stop codon is located in the *TPR1* promoter, resulting in an extension of 18 amino acids.
Figure 4 sets out a schematic representation of how UGT2 is integrated into the genome. A. different fragments used in transformation; B. situation after integration; C. situation after expression of Cre recombinase).
Figure 5 sets out a schematic representation of how the pathway from GGPP to Steviol is integrated into the genome. A. different fragments used in transformation; B. situation after integration.
Figure 6 sets out a schematic representation of how the pathway from GGPP to RebA is integrated into the genome. A. different fragments used in transformation; B. situation after integration.
Figure 7 sets out steviol production in strain STV018. Samples from shake flasks were taken after 7 days, treated with heat and acetonitrile, and Steviol concentrations were determined with LC/MS.
Figure 8 sets out RebA production in strain STV006. Samples from shake flask were taken after 7 days, treated with heat and acetonitrile, and Steviol concentrations were determined with LC/MS.
Figure 9 sets out RebA production in strains STV006, STV012, STV016 and STV017. Samples from shake flasks were taken after 7 days, treated with heat and acetonitrile, and RebA concentrations were determined with LC/MS.
Figure 10 sets out stevioside and RebA production in strains STV018, STV019 and STV020. Samples from shake flasks were taken after 7 days, treated with heat and acetonitrile, and stevioside and RebA concentrations were determined with LC/MS.
Figure 11 sets out a schematic representation of the plasmid MB6754.
Figure 12 sets out a schematic representation of the plasmid MB6761.
Figure 13 sets out a schematic representation of the plasmid MB6762.
Figure 14 sets out a schematic representation of the plasmid MB6775.
Figure 15 sets out a schematic representation of how the pathway from GGPP to RebD is integrated into the genome. A. different fragments used in transformation; B. situation after integration.
Figure 16 sets out RebD and RebA production in strains STV006 and STV015. Samples from shake flasks were taken after 7 days, treated with heat and acetonitrile, and RebD and RebA concentrations were determined with LC/MS.
Figure 17 sets out a schematic diagram of the potential pathways leading to biosynthesis of steviol glycosides.

### Description of the sequence listing

A description of the sequences is set out in Table 1. Sequences described herein may be defined with reference to the sequence listing or with reference to the database accession numbers also set out in Table 1.

### Detailed description

Throughout the present specification and the accompanying claims, the words "comprise", "include" and "having" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

The disclosure relates to a recombinant microorganism that is capable of producing a diterpene or a glycosylated diterpene, typically steviol or a steviol glycoside respectively. For the purposes of this disclosure, a diterpene typically means an organic compound composed of four isoprene units. Such a compound may be derived from geranylgeranyl pyrophosphate. A glycosylated diterpene or diterpene glycoside is a diterpene in which a sugar is bound, typically to a non-carbohydrate moiety. Typically, in a diterpene glycoside, the sugar group may be bonded through its anomeric carbon to another group via a glycosidic bond. A preferred diterpene and diterpene glycoside is steviol and steviol glycoside respectively. Thus, in particular, the disclosure relates to a recombinant microorganism which is capable of producing steviol or a steviol glycoside.

According to the invention, there is provided a recombinant microorganism. The recombinant microorganism which is a *Yarrowia,* such as *Yarrowia lipolytica,* comprises one or more nucleotide sequence(s) encoding:
a polypeptide having ent-copalyl pyrophosphate synthase activity;
a polypeptide having ent-Kaurene synthase activity;
a polypeptide having ent-Kaurene oxidase activity; and
a polypeptide having kaurenoic acid 13-hydroxylase activity,
a polypeptide having NADPH-cytochrome p450 reductase activity; and
a polypeptide having UDP-glucosyltransferase activity,
wherein a nucleotide sequence encoding a polypeptide having ent-Kaurene oxidase activity encodes a polypeptide comprising an amino acid sequence that has at least 20% sequence identity with the amino acid sequence of SEQ ID NO: 86; and
wherein a nucleotide sequence encoding a polypeptide having kaurenoic acid 13-hydroxylase activity encodes a polypeptide comprising an amino acid sequence that has at least 20% sequence identity with the amino acid sequence of SEQ ID NO: 34,
whereby expression of the nucleotide sequence(s) confer(s) on the microorganism the ability to produce at least one of steviolmonoside, steviolbioside, stevioside or rebaudioside A, rebaudioside B, rebaudioside C, rebaudioside D, rebaudioside E, rebaudioside F, rubusoside, dulcoside A.

For the purposes of this disclosure, a polypeptide having *ent*-copalyl pyrophosphate synthase (EC 5.5.1.13) is capable of catalyzing the chemical reation:

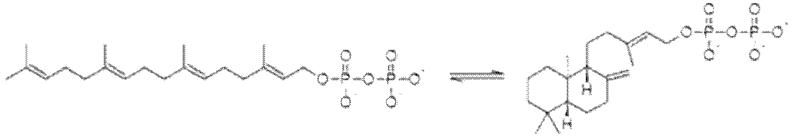

This enzyme has one substrate, geranylgeranyl pyrophosphate, and one product, *ent*-copalyl pyrophosphate. This enzyme participates in gibberellin biosynthesis. This enzyme belongs to the family of isomerase, specifically the class of intramolecular lyases. The systematic name of this enzyme class is *ent*-copalyl-diphosphate lyase (decyclizing). Other names in common use include having *ent*-copalyl pyrophosphate synthase, *ent-*kaurene synthase A, and *ent*-kaurene synthetase A.

For the purposes of this disclosure, a polypeptide having ent-kaurene synthase activity (EC 4.2.3.19) is a polypeptide that is capable of catalyzing the chemical reaction:

ent-copalyl diphosphate ⇄ent-kaurene + diphosphate

Hence, this enzyme has one substrate, ent-copalyl diphosphate, and two products, ent-kaurene and diphosphate.

This enzyme belongs to the family of lyases, specifically those carbon-oxygen lyases acting on phosphates. The systematic name of this enzyme class is ent-copalyl-diphosphate diphosphate-lyase (cyclizing, ent-kaurene-forming). Other names in common use include ent-kaurene synthase B, ent-kaurene synthetase B, ent-copalyl-diphosphate diphosphate-lyase, and (cyclizing). This enzyme participates in diterpenoid biosynthesis.
*ent*-copalyl diphosphate synthases may also have a distinct *ent*-kaurene synthase activity associated with the same protein molecule. The reaction catalyzed by *ent*-kaurene synthase is the next step in the biosynthetic pathway to gibberellins. The two types of enzymic activity are distinct, and site-directed mutagenesis to suppress the *ent*-kaurene synthase activity of the protein leads to build up of *ent*-copalyl pyrophosphate.

Accordingly, a single nucleotide sequence used in the disclosure may encode a polypeptide having *ent*-copalyl pyrophosphate synthase activity and *ent*-kaurene synthase activity. Alternatively, the two activities may be encoded two distinct, separate nucleotide sequences.

For the purposes of this disclosure, a polypeptide having ent-kaurene oxidase activity (EC 1.14.13.78) is a polypeptide which is capable of catalysing three successive oxidations of the 4-methyl group of ent-kaurene to give kaurenoic acid. Such activity typically requires the presence of a cytochrome P450.

For the purposes of the disclosure, a polypeptide having kaurenoic acid 13-hydroxylase activity (EC 1.14.13) is one which is capable of catalyzing the formation of steviol (ent-kaur-16-en-13-ol-19-oic acid) using NADPH and O₂. Such activity may also be referred to as ent-ka 13-hydroxylase activity.

A recombinant microorganism of the invention comprises one or more nucleotide sequences encoding a polypeptide having UDP-glucosyltransferase (UGT) activity, whereby expression of the nucleotide sequence(s) confer(s) on the microorganism the ability to produce at least one of steviolmonoside, steviolbioside, stevioside or rebaudioside A, rebaudioside B, rebaudioside C, rebaudioside D, rebaudioside E, rebaudioside F, rubusoside, dulcoside A.

For the purposes of this disclosure, a polypeptide having UGT activity is one which has glycosyltransferase activity (EC 2.4), i.e. that can act as a catalyst for the transfer of a monosaccharide unit from an activated nucleotide sugar (also known as the "glycosyl donor") to a glycosyl acceptor molecule, usually an alcohol. The glycosyl donor for a UGT is typically the nucleotide sugar uridine diphosphate glucose (uracil-diphosphate glucose, UDP-glucose).

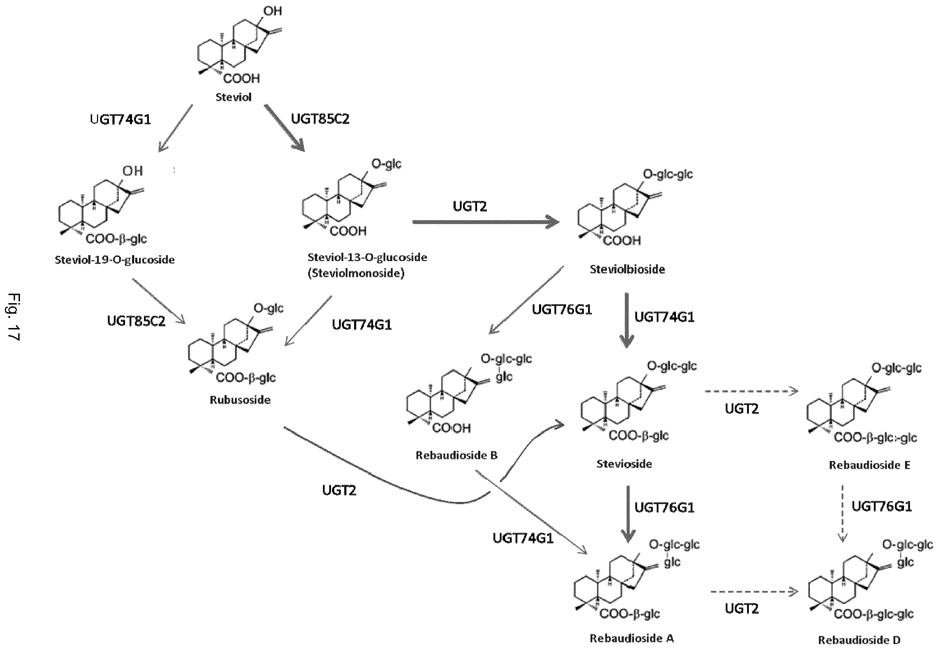

The UGTs used may be selected so as to produce a desired diterpene glycoside, such as a steviol glycoside. Schematic diagrams of steviol glycoside formation are set out in Humphrey et al., Plant Molecular Biology (2006) 61: 47-62 and Mohamed et al., J. Plant Physiology 168 (2011) 1136-1141. In addition, Figure 17 sets out a schematic diagram of steviol glycoside formation.

The biosynthesis of rebaudioside A involves glucosylation of the aglycone steviol. Specifically, rebaudioside A can be formed by glucosylation of the 13-OH of steviol which forms the 13-*O*-steviolmonoside, glucosylation of the C-2' of the 13-O-glucose of steviolmonoside which forms steviol-1,2-bioside, glucosylation of the C-19 carboxyl of steviol-1,2-bioside which forms stevioside, and glucosylation of the C-3' of the C-13-O-glucose of stevioside. The order in which each glucosylation reaction occurs can vary - see Figure 17. One UGT may be capable of catalyzing more than one conversion as set out in this scheme.

We have shown that conversion of steviol to rebaudioside A or rebaudioside D may be accomplished in a recombinant host by the expression of gene(s) encoding the following functional UGTs: UGT74G1, UGT85C2, UGT76G1 and UGT2. Thus, a recombinant microorganism expressing these four UGTs can make rebaudioside A if it produces steviol or when fed steviol in the medium. Typically, one or more of these genes are recombinant genes that have been transformed into a microorganism that does not naturally possess them. Examples of all of these enzmyes are set out in Table 1. A microorganism of the invention may comprise any combination of a UGT74G1, UGT85C2, UGT76G1 and UGT2. In Table 1 UGT64G1 sequences are indicated as UGT1 sequences, UGT74G1 sequences are indicated as UGT3 sequences and UGT76G1 sequences are indicated as UGT4 sequences. UGT2 sequences are indicated as UGT2 sequences in Table 1.

A recombinant microorganism of the invention which comprises a nucleotide sequence encoding a polypeptide having UGT activity may comprise a nucleotide sequence encoding a polypeptide capable of catalyzing the addition of a C-13-glucose to steviol. That is to say, a microorganism of the invention may comprise a UGT which is capable of catalyzing a reaction in which steviol is converted to steviolmonoside. Accordingly, expression of such a nucleotide sequence may confer on the microorganism the ability to produce at least steviolmonoside.

Such a microorganism of the invention may comprise a nucleotide sequence encoding a polypeptide having the activity shown by UDP-glycosyltransferase (UGT) UGT85C2, whereby the nucleotide sequence upon transformation of the microorganism confers on the cell the ability to convert steviol to steviolmonoside.

UGT85C2 activity is transfer of a glucose unit to the 13-OH of steviol. Thus, a suitable UGT85C2 may function as a uridine 5'-diphospho glucosyl: steviol 13-OH transferase, and a uridine 5'-diphospho glucosyl: steviol- 19-0- glucoside 13-OH transferase. A functional UGT85C2 polypeptides may also catalyze glucosyl transferase reactions that utilize steviol glycoside substrates other than steviol and steviol- 19-O-glucoside. Such sequences are indicated as UGT1 sequences in Table 1.

A recombinant microorganism of the invention which comprises a nucleotide sequence encoding a polypeptide having UGT activity may comprise a nucleotide sequence encoding a polypeptide capable of catalyzing the addition of a C-13-glucose to steviol or steviolmonoside. That is to say, a microorganism of the invention may comprise a UGT which is capable of catalyzing a reaction in which steviolmonoside is converted to steviolbioside. Accordingly, such a microorganism may be capable of converting steviolmonoside to steviolbioside. Expression of such a nucleotide sequence may confer on the microorganism the ability to produce at least steviolbioside.

A microorganism of the invention may also comprise a nucleotide sequence encoding a polypeptide having the activity shown by UDP-glycosyltransferase (UGT) UGT74G1, whereby the nucleotide sequence upon transformation of the microorganism confers on the cell the ability to convert steviolmonoside to steviolbioside.

A microorganism of the invention may also comprise a nucleotide sequence encoding a polypeptide having the activity shown by UDP-glycosyltransferase (UGT) UGT2, whereby the nucleotide sequence upon transformation of the microorganism confers on the cell the ability to convert steviolmonoside to steviolbioside.

A suitable UGT2 polypeptide functions as a uridine 5'-diphospho glucosyl: steviol- 13-0-glucoside transferase (also referred to as a steviol-13- monoglucoside 1,2-glucosylase), transferring a glucose moiety to the C-2' of the 13- O-glucose of the acceptor molecule, steviol-13-O-glucoside. Typically, a suitable UGT2 polypeptide also functions as a uridine 5'-diphospho glucosyl: rubusoside transferase transferring a glucose moiety to the C-2' of the 13-O-glucose of the acceptor molecule, rubusoside.

Functional UGT2 polypeptides may also catalyze reactions that utilize steviol glycoside substrates other than steviol- 13-0-glucoside and rubusoside, e.g., functional UGT2 polypeptides may utilize stevioside as a substrate, transferring a glucose moiety to the C-2' of the 19-0-glucose residue to produce Rebaudioside E. A functional UGT2 polypeptides may also utilize Rebaudioside A as a substrate, transferring a glucose moiety to the C-2' of the 19-O-glucose residue to produce Rebaudioside D. However, a functional UGT2 polypeptide typically does not transfer a glucose moiety to steviol compounds having a 1,3-bound glucose at the C- 13 position, i.e., transfer of a glucose moiety to steviol 1,3-bioside and 1,3-stevioside does not occur.
Functional UGT2 polypeptides may also transfer sugar moieties from donors other than uridine diphosphate glucose. For example, a functional UGT2 polypeptide may act as a uridine 5'-diphospho D-xylosyl: steviol- 13 -O-glucoside transferase, transferring a xylose moiety to the C-2' of the 13-O-glucose of the acceptor molecule, steviol- 13 -O-glucoside. As another example, a functional UGT2 polypeptide can act as a uridine 5'-diphospho L-rhamnosyl: steviol- 13-0-glucoside transferase, transferring a rhamnose moiety to the C-2' of the 13-O-glucose of the acceptor molecule, steviol-13-O-glucoside. Such sequences are indicated as UGT2 sequences in Table 1.

A recombinant microorganism of the invention which comprises a nucleotide sequence encoding a polypeptide having UGT activity may comprise a nucleotide sequence encoding a polypeptide capable of catalyzing the addition of a C-19-glucose to steviolbioside. That is to say, a microorganism of the invention may comprise a UGT which is capable of catalyzing a reaction in which steviolbioside is converted to stevioside. Accordingly, such a microorganism may be capable of converting steviolbioside to stevioside. Expression of such a nucleotide sequence may confer on the microorganism the ability to produce at least stevioside.

A microorganism of the invention may also comprise a nucleotide sequence encoding a polypeptide having the activity shown by UDP-glycosyltransferase (UGT) UGT74G1, whereby the nucleotide sequence upon transformation of the microorganism confers on the cell the ability to convert steviolbioside to stevioside.

Suitable UGT74G1 polypeptides may be capable of transferring a glucose unit to the 13-OH or the 19-COOH, respectively, of steviol. A suitable UGT74G1 polypeptide may function as a uridine 5'-diphospho glucosyl: steviol 19-COOH transferase and a uridine 5'-diphospho glucosyl: steviol- 13-O-glucoside 19-COOH transferase. Functional UGT74G1 polypeptides also may catalyze glycosyl transferase reactions that utilize steviol glycoside substrates other than steviol and steviol- 13-O-glucoside, or that transfer sugar moieties from donors other than uridine diphosphate glucose. Such sequences are indicated as UGT1 sequences in Table 3.

A recombinant microorganism of the invention which comprises a nucleotide sequence encoding a polypeptide having UGT activity may comprise a nucleotide sequence encoding a polypeptide capable of catalyzing glucosylation of the C-3' of the glucose at the C-13 position of stevioside. That is to say, a microorganism of the invention may comprise a UGT which is capable of catalyzing a reaction in which stevioside to rebaudioside A. Accordingly, such a microorganism may be capable of converting stevioside to rebaudioside A. Expression of such a nucleotide sequence may confer on the microorganism the ability to produce at least rebaudioside A.

A microorganism of the invention may also comprise a nucleotide sequence encoding a polypeptide having the activity shown by UDP-glycosyltransferase (UGT) UGT76G1, whereby the nucleotide sequence upon transformation of the microorganism confers on the cell the ability to convert stevioside to rebaudioside A.

A suitable UGT76G1 adds a glucose moiety to the C-3'of the C-13-O-glucose of the acceptor molecule, a steviol 1,2 glycoside. Thus, UGT76G1 functions, for example, as a uridine 5'-diphospho glucosyl: steviol 13-0-1,2 glucoside C-3 ' glucosyl transferase and a uridine 5'-diphospho glucosyl: steviol- 19-0-glucose, 13-0-1,2 bioside C-3' glucosyl transferase. Functional UGT76G1 polypeptides may also catalyze glucosyl transferase reactions that utilize steviol glycoside substrates that contain sugars other than glucose, e.g., steviol rhamnosides and steviol xylosides. Such sequences are indicated as UGT4 sequences in Table 1.

A microorganism of the invention may comprise nucleotide sequences encoding polypeptides having one or more of the four UGT activities described above. Preferably, a microorganism of the invention may comprise nucleotide sequences encoding polypeptides having all four of the UGT activities described above. A given nucleic acid may encode a polypeptide having one or more of the above activities. For example, a nucleic acid encode for a polypeptide which has two, three or four of the activities set out above. Preferably, a recombinant microorganism of the invention comprises UGT1, UGT2 and UGT3 activity. More preferably, such a recombinant microorganism will also comprise UGT4 activity.

A microorganism of the invention which comprises a nucleotide sequence encoding a polypeptide having UGT activity may comprise a nucleotide sequence encoding a polypeptide capable of catalyzing the glucosylation of stevioside or rebaudioside A. That is to say, a microorganism of the invention may comprise a UGT which is capable of catalyzing a reaction in which stevioside or rebaudioside A is converted to rebaudioside D. Accordingly, such a microorganism may be capable of converting stevioside or rebaudioside A to rebaudioside D. Expression of such a nucleotide sequence may confer on the microorganism the ability to produce at least rebaudioside D. We have shown that a microorganism expression a combination of UGT85C2, UGT2, UGT74G1 and UGT76G1 polypeptides may be capable of rebaudioside D production.

A microorganism of the invention which comprises a nucleotide sequence encoding a polypeptide having UGT activity may comprise a nucleotide sequence encoding a polypeptide capable of catalyzing the glucosylation of stevioside. That is to say, a microorganism of the invention may comprise a UGT which is capable of catalyzing a reaction in which stevioside is converted to rebaudioside E. Accordingly, such a microorganism may be capable of converting stevioside to rebaudioside E. Expression of such a nucleotide sequence may confer on the microorganism the ability to produce at least rebaudioside E.

A microorganism of the invention which comprises a nucleotide sequence encoding a polypeptide having UGT activity may comprise a nucleotide sequence encoding a polypeptide capable of catalyzing the glucosylation of rebaudioside E. That is to say, a microorganism of the invention may comprise a UGT which is capable of catalyzing a reaction in which rebaudioside E is converted to rebaudioside D. Accordingly, such a microorganism may be capable of converting stevioside or rebaudioside A to rebaudioside D. Expression of such a nucleotide sequence may confer on the microorganism the ability to produce at least rebaudioside D.

A recombinant microorganism of the invention is capable of expressing a nucleotide sequence encoding a polypeptide having NADPH-cytochrome p450 reductase activity. That is to say, a recombinant microorganism of the invention comprises sequence encoding a polypeptide having NADPH-cytochrome p450 reductase activity.

For the purposes of the disclosure, a polypeptide having NADPH-Cytochrome P450 reductase activity (EC 1.6.2.4; also known as NADPH:ferrihemoprotein oxidoreductase, NADPH:hemoprotein oxidoreductase, NADPH:P450 oxidoreductase, P450 reductase, POR, CPR, CYPOR) is typically one which is a membrane-bound enzyme allowing electron transfer to cytochrome P450 in the microsome of the eukaryotic cell from a FAD- and FMN-containing enzyme NADPH:cytochrome P450 reductase (POR; EC 1.6.2.4).

Preferably, a recombinant microorganism according to the disclosure is capable of expressing one or more of:
a. a nucleotide sequence encoding a polypeptide having NADPH-cytochrome p450 reductase activity, wherein said nucleotide sequence comprises:
   i. a nucleotide sequence encoding a polypeptide having NADPH-cytochrome p450 reductase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 54, 56, 58 or 78;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 53, 55, 57 or 77;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code,

Preferably, a recombinant microorganism of the disclosure is one which is capable of expressing one or more of:
a. a nucleotide sequence encoding a polypeptide having ent-copalyl pyrophosphate synthase activity, wherein said nucleotide sequence comprises:
   i. a nucleotide sequence encoding a polypeptide having ent-copalyl pyrophosphate synthase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 2, 4, 6, 8, 18, 20, 60 or 62;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 1, 3, 5, 7, 17, 19, 59 or 61, 141, 142, 151, 152, 153, 154, 159, 160, 182 or 184;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code,
b. a nucleotide sequence encoding a polypeptide having ent-Kaurene synthase activity, wherein said nucleotide sequence comprises:
   i. a nucleotide sequence encoding a polypeptide having ent-Kaurene synthase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 10, 12, 14, 16, 18, 20, 64 or 66;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 9, 11, 13, 15, 17, 19, 63, 65, 143, 144, 155, 156, 157, 158, 159, 160, 183 or 184;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code,
c. a nucleotide sequence encoding a polypeptide having ent-Kaurene oxidase activity, wherein said nucleotide sequence comprises:
   i. a nucleotide sequence encoding a polypeptide having ent-Kaurene oxidase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 22, 24, 26, 68 or 86;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 21, 23, 25, 67, 85, 145, 161, 162, 163, 180 or 186;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code; or
d. a nucleotide sequence encoding a polypeptide having kaurenoic acid 13-hydroxylase activity, wherein said nucleotide sequence comprises:
   i. a nucleotide sequence encoding a polypeptide having kaurenoic acid 13-hydroxylase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 28, 30, 32, 34, 70, 90, 92, 94, 96 or 98;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 27, 29, 31, 33, 69, 89, 91, 93, 95, 97, 146, 164, 165, 166, 167 or 185;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code.

In a recombinant microorganism of the disclosure, which is capable of expressing a nucleotide sequence encoding a polypeptide capable of catalyzing the addition of a C-13-glucose to steviol, said nucleotide may comprise:
i. a nucleotide sequence encoding a polypeptide capable of catalyzing the addition of a C-13-glucose to steviol, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 36, 38 or 72;
ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 35, 37, 71, 147, 168, 169 or 189;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

In a recombinant microorganism of the disclosure, which is capable of expressing a nucleotide sequence encoding a polypeptide capable of catalyzing the addition of a glucose at the C-13 position of steviolmonoside (this typically indicates glucosylation of the C-2' of the C-13-glucose/13-O-glucose of steviolmonoside), said nucleotide sequence may comprise:
i. a nucleotide sequence encoding a polypeptide capable of catalyzing the addition of a C-13-glucose to steviol or steviolmonoside, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 88, 100, 102, 104, 106, 108, 110 or 112;
ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 87, 99, 101, 103, 105, 107, 109, 111, 181 or 192;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

In a recombinant microorganism of the disclosure, which is capable of expressing a nucleotide sequence encoding a polypeptide capable of catalyzing the addition of a glucose at the C-19 position of steviolbioside, said nucleotide sequence may comprise:
i. a nucleotide sequence encoding a polypeptide capable of catalyzing the addition of a glucose at the C-19 position of steviolbioside, said polypeptide comprising an amino acid sequence that has at least about 20% sequence identity with the amino acid sequence of SEQ ID NOs: 40, 42, 44, 46, 48 or 74;
ii. a nucleotide sequence that has at least about 15% sequence identity with the nucleotide sequence of SEQ ID NOs: 39, 41, 43, 45, 47, 73, 148, 170, 171, 172, 173, 174 or 190 ;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

In a recombinant microorganism of the disclosure, which expresses a nucleotide sequence encoding a polypeptide capable of catalyzing glucosylation of the C-3' of the glucose at the C-13 position of stevioside, said nucleotide sequence may comprise:
i. a nucleotide sequence encoding a polypeptide capable of catalyzing glucosylation of the C-3' of the glucose at the C-13 position of stevioside, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 50, 52 or 76;
ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 49, 51, 75, 149, 175, 176 or 191;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

In a recombinant microorganism of the disclosure, which expresses a nucleotide sequence encoding a polypeptide capable of catalysing one or more of: the glucosylation of stevioside or rebaudioside A to rebaudioside D; the glucosylation of stevioside to rebaudioside E; or the glucosylation of rebaudioside E to rebaudioside D, said nucleotide sequence may comprise:
i. a nucleotide sequence encoding a polypeptide capable of catalysing one or more of: the glucosylation of stevioside or rebaudioside A to rebaudioside D; the glucosylation of stevioside to rebaudioside E; or the glucosylation of rebaudioside E to rebaudioside D, said polypeptide comprising an amino acid sequence that has at least about 20% sequence identity with the amino acid sequence of SEQ ID NOs: 88, 100, 102, 104, 106, 108, 110, 112;
ii. a nucleotide sequence that has at least about 15% sequence identity with the nucleotide sequence of SEQ ID NOs: 87, 99, 101, 103, 105, 107, 109, 111, 181 or 192;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

A microorganism according to the invention, may be one in which the ability of the microorganism to produce geranylgeranyl pyrophosphate (GGPP) is upregulated. Upregulated in the context of this disclosure implies that the microorganism produces more GGPP than an equivalent non-transformed strain.

Accordingly, a microorganism of the invention may comprise one or more nucleotide sequence(s) encoding hydroxymethylglutaryl-CoA reductase, farnesyl-pyrophosphate synthetase and geranylgeranyl diphosphate synthase, whereby the nucleotide sequence(s) upon transformation of the microorganism confer(s) on the microorganism the ability to produce elevated levels of GGPP.

Preferably, a microorganism according to the disclosure is one which is capable of expressing one or more of:
a. a nucleotide sequence encoding a polypeptide having hydroxymethylglutaryl-CoA reductase activity, wherein said nucleotide sequence comprises:
   i. a nucleotide sequence encoding a polypeptide having hydroxymethylglutaryl-CoA reductase activity, said polypeptide comprising an amino acid sequence that has at least about 20% sequence identity with the amino acid sequence of SEQ ID NO: 80;
   ii. a nucleotide sequence that has at least about 15% sequence identity with the nucleotide sequence of SEQ ID NO: 79;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code,
b. a nucleotide sequence encoding a polypeptide having farnesyl-pyrophosphate synthetase activity, wherein said nucleotide sequence comprises:
   i. a nucleotide sequence encoding a polypeptide having farnesyl-pyrophosphate synthetase activity, said polypeptide comprising an amino acid sequence that has at least about 20% sequence identity with the amino acid sequence of SEQ ID NO: 82;
   ii. a nucleotide sequence that has at least about 15% sequence identity with the nucleotide sequence of SEQ ID NOs: 81;
   iii. a nucleotide sequence the complementary strand of which hybridizes to a nucleic acid molecule of sequence of (i) or (ii); or
   iv. a nucleotide sequence which differs from the sequence of a nucleic acid molecule of (iii) due to the degeneracy of the genetic code; or
c. a nucleotide sequence encoding a polypeptide having geranylgeranyl diphosphate synthase activity, wherein said nucleotide sequence comprises:
   i. a nucleotide sequence encoding a polypeptide having geranylgeranyl diphosphate synthase activity, said polypeptide comprising an amino acid sequence that has at least about 20% sequence identity with the amino acid sequence of SEQ ID NO: 84;
   ii. a nucleotide sequence that has at least about 15% sequence identity with the nucleotide sequence of SEQ ID NOs: 83;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code.

The disclosure relates to a recombinant microorganism. A microorganism or microbe, for the purposes of this disclosure, is typically an organism that is not visible to the human eye (i.e. microscopic). A microorganism may be from bacteria, fungi, archaea or protists. Typically a microorganism will be a single-celled or unicellular organism.

As used herein a recombinant microorganism is defined as a microorganism which is genetically modified or transformed/transfected with one or more of the nucleotide sequences as defined herein. The presence of the one or more such nucleotide sequences alters the ability of the microorganism to produce a diterpene or diterpene glycoside, in particular steviol or steviol glycoside. A microorganism that is not transformed/transfected or genetically modified, is not a recombinant microorganism and does typically not comprise one or more of the nucleotide sequences enabling the cell to produce a diterpene or diterpene glycoside. Hence, a non-transformed/non-transfected microorganism is typically a microorganism that does not naturally produce a diterpene, although a microorganism which naturally produces a diterpene or diterpene glycoside and which has been modified according to the disclosure (and which thus has an altered ability to produce a diterpene/diterpene gylcoside) is considered a recombinant microorganism according to the disclosure.

Sequence identity is herein defined as a relationship between two or more amino acid (polypeptide or protein) sequences or two or more nucleic acid (polynucleotide) sequences, as determined by comparing the sequences. Usually, sequence identities or similarities are compared over the whole length of the sequences compared. In the art, "identity" also means the degree of sequence relatedness between amino acid or nucleic acid sequences, as the case may be, as determined by the match between strings of such sequences. "Identity" and "similarity" can be readily calculated by various methods, known to those skilled in the art. Preferred methods to determine identity are designed to give the largest match between the sequences tested. Typically then, identities and similarities are calculated over the entire length of the sequences being compared. Methods to determine identity and similarity are codified in publicly available computer programs. Preferred computer program methods to determine identity and similarity between two sequences include e.g. the BestFit, BLASTP, BLASTN, and FASTA (Altschul, S. F. et al., J. Mol. Biol. 215:403-410 (1990), publicly available from NCBI and other sources (BLAST Manual, Altschul, S., et al., NCBI NLM NIH Bethesda, MD 20894). Preferred parameters for amino acid sequences comparison using BLASTP are gap open 10.0, gap extend 0.5, Blosum 62 matrix. Preferred parameters for nucleic acid sequences comparison using BLASTP are gap open 10.0, gap extend 0.5, DNA full matrix (DNA identity matrix).

Nucleotide sequences encoding the enzymes expressed in the cell of the disclosure may also be defined by their capability to hybridize with the nucleotide sequences of SEQ ID NO.'s 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81 or 84 ir any other sequence mentioned herein respectively, under moderate, or preferably under stringent hybridisation conditions. Stringent hybridisation conditions are herein defined as conditions that allow a nucleic acid sequence of at least about 25, preferably about 50 nucleotides, 75 or 100 and most preferably of about 200 or more nucleotides, to hybridise at a temperature of about 65°C in a solution comprising about 1 M salt, preferably 6 x SSC or any other solution having a comparable ionic strength, and washing at 65°C in a solution comprising about 0.1 M salt, or less, preferably 0.2 x SSC or any other solution having a comparable ionic strength. Preferably, the hybridisation is performed overnight, i.e. at least for 10 hours and preferably washing is performed for at least one hour with at least two changes of the washing solution. These conditions will usually allow the specific hybridisation of sequences having about 90% or more sequence identity.

Moderate conditions are herein defined as conditions that allow a nucleic acid sequences of at least 50 nucleotides, preferably of about 200 or more nucleotides, to hybridise at a temperature of about 45°C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. Preferably, the hybridisation is performed overnight, i.e. at least for 10 hours, and preferably washing is performed for at least one hour with at least two changes of the washing solution. These conditions will usually allow the specific hybridisation of sequences having up to 50% sequence identity. The person skilled in the art will be able to modify these hybridisation conditions in order to specifically identify sequences varying in identity between 50% and 90%.

The nucleotide sequences encoding an ent-copalyl pyrophosphate synthase; ent-Kaurene synthase; ent-Kaurene oxidase; kaurenoic acid 13-hydroxylase; UGT; hydroxymethylglutaryl-CoA reductase, farnesyl-pyrophosphate synthetase; geranylgeranyl diphosphate synthase; NADPH-cytochrome p450 reductase, may be from prokaryotic or eukaryotic origin.

A nucleotide sequence encoding an ent-copalyl pyrophosphate synthase may for instance comprise a sequence as set out in SEQ ID. NO: 1, 3, 5, 7, 17, 19, 59, 61, 141, 142, 151, 152, 153, 154, 159, 160, 182 or 184.

A nucleotide sequence encoding an ent-Kaurene synthase may for instance comprise a sequence as set out in SEQ ID. NO: 9, 11, 13, 15, 17, 19, 63, 65, 143, 144, 155, 156, 157, 158, 159, 160, 183 or 184.

A nucleotide sequence encoding an ent-Kaurene oxidase may for instance comprise a sequence as set out in SEQ ID. NO: 21, 23, 25, 67, 85, 145, 161, 162, 163, 180 or 186. A preferred KO is the polypeptide encoded by the nucleic acid set out in SEQ ID NO: 85.

A nucleotide sequence encoding a kaurenoic acid 13-hydroxylase may for instance comprise a sequence as set out in SEQ ID. NO: 27, 29, 31, 33, 69, 89, 91, 93, 95, 97, 146, 164, 165, 166, 167 or 185. A preferred KAH sequence is the polypeptide encoded by the nucleic acid set out in SEQ ID NO: 33.

A further preferred recombinant microorganism of the disclosure may express a combination of the polypeptides encoded by SEQ ID NO: 85 and SEQ ID NO: 33 or a variant of either thereof as herein described. A preferred recombinant microorganism of the disclosure may expression the combination of sequences set out in Table 8 (in combination with any UGT2, but in particular that encoded by SEQ ID NO: 87).

A nucleotide sequence encoding a UGT may for instance comprise a sequence as set out in SEQ ID. NO: 35, 37, 39, 41, 43, 45, 47, 49, 51, 71, 73, 75, 168, 169, 170, 171, 172, 173, 174, 175, 176, 147, 148, 149, 87, 181, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 189, 190, 191 or 192.

A nucleotide sequence encoding a hydroxymethylglutaryl-CoA reductase may for instance comprise a sequence as set out in SEQ ID. NO: 79.

A nucleotide sequence encoding a farnesyl-pyrophosphate synthetase may for instance comprise a sequence as set out in SEQ ID. NO: 81.

A nucleotide sequence encoding a geranylgeranyl diphosphate synthase may for instance comprise a sequence as set out in SEQ ID. NO:83.

A nucleotide sequence encoding a NADPH-cytochrome p450 reductase may for instance comprise a sequence as set out in SEQ ID. NO: 53, 55, 57 or 77.

In the case of the UGT sequences, combinations of at least one from each of: (i) SEQ ID NOs: 35, 37, 168, 169, 71, 147 or 189; (ii) SEQ ID NOs: 87, 99, 101, 103, 105, 107, 109, 111, 181 or 192; (iii) SEQ ID NOs: 39, 41, 43, 45, 47, 170, 171, 172, 173, 174, 73, 148 or 190; and (iv) SEQ ID NOs: 49, 51, 175, 176, 75, 149 or 191 may be preferred. Typically, at least one UGT from group (i) may be used. If at least one UGT from group (iii) is used, generally at least one UGT from group (i) is also used. If at least one UGT from group (iv) is used, generally at least one UGT from group (i) and at least one UGT from group (iii) is used. Typically, at least one UGT form group (ii) is used.

A sequence which has at least about 10%, about 15%, about 20%, preferably at least about 25%, about 30%, about 40%, about 50%, about 55%, about 60%, about 65%, about 70%, about 75%, about 80%, about 85%, about 90%, about 95%, about 96%, about 97%, about 98%, or about 99% sequence identity with a sequence as mentioned may be used in the disclosure.

To increase the likelihood that the introduced enzymes are expressed in active form in a eukaryotic cell of the disclosure, the corresponding encoding nucleotide sequence may be adapted to optimise its codon usage to that of the chosen eukaryote host cell. The adaptiveness of the nucleotide sequences encoding the enzymes to the codon usage of the chosen host cell may be expressed as codon adaptation index (CAI). The codon adaptation index is herein defined as a measurement of the relative adaptiveness of the codon usage of a gene towards the codon usage of highly expressed genes. The relative adaptiveness (w) of each codon is the ratio of the usage of each codon, to that of the most abundant codon for the same amino acid. The CAI index is defined as the geometric mean of these relative adaptiveness values. Non-synonymous codons and termination codons (dependent on genetic code) are excluded. CAI values range from 0 to 1, with higher values indicating a higher proportion of the most abundant codons (see Sharp and Li, 1987, Nucleic Acids Research 15: 1281-1295; also see: Jansen et al., 2003, Nucleic Acids Res. 31(8):2242-51). An adapted nucleotide sequence preferably has a CAI of at least 0.2, 0.3, 0.4, 0.5, 0.6 or 0.7.

In a preferred embodiment the eukaryotic cell according to the present disclosure is genetically modified with (a) nucleotide sequence(s) which is (are) adapted to the codon usage of the eukaryotic cell using codon pair optimisation technology as disclosed in PCT/EP2007/05594. Codon-pair optimisation is a method for producing a polypeptide in a host cell, wherein the nucleotide sequences encoding the polypeptide have been modified with respect to their codon-usage, in particular the codon-pairs that are used, to obtain improved expression of the nucleotide sequence encoding the polypeptide and/or improved production of the polypeptide. Codon pairs are defined as a set of two subsequent triplets (codons) in a coding sequence.

Further improvement of the activity of the enzymes *in vivo* in a eukaryotic host cell of the disclosure, can be obtained by well-known methods like error prone PCR or directed evolution. A preferred method of directed evolution is described in WO03010183 and WO03010311.

The microorganism according to the present disclosure may be any suitable host cell from microbial origin. Preferably, the host cell is a yeast or a filamentous fungus. More preferably, the host cell belongs to one of the genera *Saccharomyces, Aspergillus, Penicillium, Pichia, Kluyveromyces, Yarrowia, Candida, Hansenula, Humicola, Torulaspora, Trichosporon, Brettanomyces, Pachysolen* or *Yamadazyma* or *Zygosaccharomyces.*

A more preferred microorganism belongs to the species *Aspergillus niger, Penicillium chrysogenum, Pichia stipidis, Kluyveromyces marxianus, K. lactis, K. thermotolerans, Yarrowia lipolytica, Candida sonorensis, C. glabrata, Hansenula polymorpha, Torulaspora delbrueckii, Brettanomyces bruxellensis, Zygosaccharomyces bailii, Saccharomyces uvarum, Saccharomyces bayanus* or *Saccharomyces cerevisiae* species. Preferably, the eukaryotic cell is a *Saccharomyces cerevisiae.* The recombinant microorganism according to the invention belongs to the genus *Yarrowia,* such as to the species *Yarrowia lipolytica.*

A recombinant yeast cell according to the disclosure may be modified so that the ERG9 gene is down-regulated and or the ERG5/ERG6 genes are deleted. Corresponding genes may be modified in this way in other microorganisms.

Such a microorganism may be transformed as set out herein, whereby the nucleotide sequence(s) with which the microorganism is transformed confer(s) on the cell the ability to produce a diterpene or glycoside thereof.

A preferred microorganism according to the disclosure is a yeast such as a *Saccharomyces cerevisiae* or *Yarrowia lipolytica* cell. A recombinant microorganism of the disclosure, such as a recombinant *Saccharomyces cerevisiae* cell or *Yarrowia lipolytica* cell may comprise one or more nucleotide sequence(s) from each of the following groups;
(i) SEQ ID. NO: 1, 3, 5, 7, 17, 19, 59, 61, 141, 142, 152, 153, 154, 159, 160, 182 or 184.
(ii) SEQ ID. NO: 9, 11, 13, 15, 17, 19, 63, 65, 143, 144, 155, 156, 157, 158, 159, 160, 183 or 184.
(iii) SEQ ID. NO: 21, 23, 25, 67 85, 145, 161, 162, 163, 180 or 186.
(iv) SEQ ID. NO: 27, 29, 31, 33, 69, 89, 91, 93, 95, 97, 146, 164, 165, 166, 167 or 185.

Such a microorganism will typically also comprise one or more nucleotide sequence(s) as set out in SEQ ID. NO: 53, 55, 57 or 77.

Such a microorganism may also comprise one or more nucleotide sequences as set out in 35, 37, 39, 41, 43, 45, 47, 49, 51, 71, 73, 75, 168, 169, 170, 171, 172, 173, 174, 175, 176, 147, 148, 149, 87, 181, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 189, 190, 191 or 192. In the case of these sequences, combinations of at least one from each of (i) SEQ ID NOs: 35, 37, 168, 169, 71, 147 or 189; (ii) SEQ ID NOs: 87, 99, 101, 103, 105, 107, 109, 111, 181 or 192; (iii) SEQ ID NOs: 39, 41, 43, 45, 47, 170, 171, 172, 173, 174, 73, 148 or 190; and (iv) SEQ ID NOs: 49, 51, 175, 176, 75, 149 or 191 may be preferred. Typically, at least one UGT from group (i) may be used. If at least one UGT from group (iii) is used, generally at least one UGT from group (i) is also used. If at least one UGT from group (iv) is used, generally at least one UGT from group (i) and at least one UGT from group (iii) is used. Typically, at least one UGT form group (ii) is used.

Such a microorganism may also comprise the following nucleotide sequences: SEQ ID. NO: 79; SEQ ID. NO: 81; and SEQ ID. NO: 83.

For each sequence set out above (or any sequence mentioned herein), a variant having at least about 15%, preferably at least about 20, about 25, about 30, about 40, about 50, about 55, about 60, about 65, about 70, about 75, about 80, about 85, about 90, about 95, about 96, about 97, about 98, or about 99%, sequence identity with the stated sequence may be used.

The nucleotide sequences encoding the ent-copalyl pyrophosphate synthase, ent-Kaurene synthase, ent-Kaurene oxidase, kaurenoic acid 13-hydroxylase, UGTs, hydroxymethylglutaryl-CoA reductase, farnesyl-pyrophosphate synthetase, geranylgeranyl diphosphate synthase and NADPH-cytochrome p450 reductase may be ligated into one or more nucleic acid constructs to facilitate the transformation of the microorganism according to the present disclosure.

A nucleic acid construct may be a plasmid carrying the genes encoding enzymes of the diterpene, eg. steviol/steviol glycoside, pathway as described above, or a nucleic acid construct may comprise two or three plasmids carrying each three or two genes, respectively, encoding the enzymes of the diterpene pathway distributed in any appropriate way.

Any suitable plasmid may be used, for instance a low copy plasmid or a high copy plasmid.

It may be possible that the enzymes selected from the group consisting of ent-copalyl pyrophosphate synthase, ent-Kaurene synthase, ent-Kaurene oxidase, and kaurenoic acid 13-hydroxylase, UGTs, hydroxymethylglutaryl-CoA reductase, farnesyl-pyrophosphate synthetase, geranylgeranyl diphosphate synthase and NADPH-cytochrome p450 reductase are native to the host microorganism and that transformation with one or more of the nucleotide sequences encoding these enzymes may not be required to confer the host cell the ability to produce a diterpene or diterpene glycosidase. Further improvement of diterpene/diterpene glycosidase production by the host microorganism may be obtained by classical strain improvement.

The nucleic acid construct may be maintained episomally and thus comprise a sequence for autonomous replication, such as an autosomal replication sequence sequence. If the host cell is of fungal origin, a suitable episomal nucleic acid construct may e.g. be based on the yeast 2µ or pKD1 plasmids (Gleer et al., 1991, Biotechnology 9: 968-975), or the AMA plasmids (Fierro et al., 1995, Curr Genet. 29:482-489).

Alternatively, each nucleic acid construct may be integrated in one or more copies into the genome of the host cell. Integration into the host cell's genome may occur at random by non-homologous recombination but preferably the nucleic acid construct may be integrated into the host cell's genome by homologous recombination as is well known in the art (see e.g. WO90/14423, EP-A-0481008, EP-A-0635 574 and US 6,265,186).

Optionally, a selectable marker may be present in the nucleic acid construct. As used herein, the term "marker" refers to a gene encoding a trait or a phenotype which permits the selection of, or the screening for, a microorganism containing the marker. The marker gene may be an antibiotic resistance gene whereby the appropriate antibiotic can be used to select for transformed cells from among cells that are not transformed. Alternatively or also, non-antibiotic resistance markers are used, such as auxotrophic markers (URA3, TRP1, LEU2). The host cells transformed with the nucleic acid constructs may be marker gene free. Methods for constructing recombinant marker gene free microbial host cells are disclosed in EP-A-0 635 574 and are based on the use of bidirectional markers. Alternatively, a screenable marker such as Green Fluorescent Protein, *lac*Z, luciferase, chloramphenicol acetyltransferase, beta-glucuronidase may be incorporated into the nucleic acid constructs of the disclosure allowing to screen for transformed cells. A preferred marker-free method for the introduction of heterologous polynucleotides is described in WO0540186.

In a preferred embodiment, the nucleotide sequences encoding ent-copalyl pyrophosphate synthase, ent-Kaurene synthase, ent-Kaurene oxidase, and kaurenoic acid 13-hydroxylase, UGTs, hydroxymethylglutaryl-CoA reductase, farnesyl-pyrophosphate synthetase, geranylgeranyl diphosphate synthase and NADPH-cytochrome p450 reductase, are each operably linked to a promoter that causes sufficient expression of the corresponding nucleotide sequences in the eukaryotic cell according to the present disclosure to confer to the cell the ability to produce a diterpene or diterpene glycoside.

As used herein, the term "operably linked" refers to a linkage of polynucleotide elements (or coding sequences or nucleic acid sequence) in a functional relationship. A nucleic acid sequence is "operably linked" when it is placed into a functional relationship with another nucleic acid sequence. For instance, a promoter or enhancer is operably linked to a coding sequence if it affects the transcription of the coding sequence.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skilled in the art to act directly or indirectly to regulate the amount of transcription from the promoter. A "constitutive" promoter is a promoter that is active under most environmental and developmental conditions. An "inducible" promoter is a promoter that is active under environmental or developmental regulation.

The promoter that could be used to achieve the expression of the nucleotide sequences coding for an enzyme as defined herein above, may be not native to the nucleotide sequence coding for the enzyme to be expressed, i.e. a promoter that is heterologous to the nucleotide sequence (coding sequence) to which it is operably linked. Preferably, the promoter is homologous, i.e. endogenous to the host cell

Suitable promoters in microorganisms of the disclosure may be GAL7, GAL10, or GAL 1, CYC1, HIS3, ADH1, PGL, PH05, GAPDH, ADC1, TRP1, URA3, LEU2, ENO, TPI, and AOX1. Other suitable promoters include PDC, GPD1, PGK1, TEF1, and TDH. Further suitable promoters are set out in the Examples.

Any terminator, which is functional in the cell, may be used in the present disclosure. Preferred terminators are obtained from natural genes of the host cell. Suitable terminator sequences are well known in the art. Preferably, such terminators are combined with mutations that prevent nonsense mediated mRNA decay in the host cell of the disclosure (see for example: Shirley et al., 2002, Genetics 161:1465-1482).

Nucleotide sequences used in the disclosure may include sequences which target them to desired compartments of the microorganism. For example, in a preferred microorganism of the disclosure, all nucleotide sequences, except for ent-Kaurene oxidase, kaurenoic acid 13-hydroxylase and NADPH-cytochrome p450 reductase encoding sequences may be targeted to the cytosol. This approach may be used in a yeast cell.

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain.

The term "heterologous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous nucleic acids or proteins are not endogenous to the cell into which it is introduced, but have been obtained from another cell or synthetically or recombinantly produced.

Typically, recombinant microorganism of the disclosure will comprise heterologous nucleotide sequences. Alternatively, a recombinant microorganism of the disclosure may comprise entirely homologous sequence which has been modified as set out herein so that the microorganism produces increased amounts of a diterpene and/or diterpene glycoside in comparison to a non-modified version of the same microorganism.

One or more enzymes of the diterpene pathway as described herein may be overexpressed to achieve a sufficient diterpene production by the cell.

There are various means available in the art for overexpression of enzymes in the host cells of the disclosure. In particular, an enzyme may be overexpressed by increasing the copy number of the gene coding for the enzyme in the host cell, e.g. by integrating additional copies of the gene in the host cell's genome.

A preferred host cell according to the present disclosure may be a recombinant cell which is naturally capable of producing GGPP.

A recombinant microorganism according to the present disclosure may be able to grow on any suitable carbon source known in the art and convert it to a diterpene or a diterpene glycoside. The recombinant microorganism may be able to convert directly plant biomass, celluloses, hemicelluloses, pectines, rhamnose, galactose, fucose, maltose, maltodextrines, ribose, ribulose, or starch, starch derivatives, sucrose, lactose and glycerol. Hence, a preferred host organism expresses enzymes such as cellulases (endocellulases and exocellulases) and hemicellulases (e.g. endo- and exo-xylanases, arabinases) necessary for the conversion of cellulose into glucose monomers and hemicellulose into xylose and arabinose monomers, pectinases able to convert pectines into glucuronic acid and galacturonic acid or amylases to convert starch into glucose monomers. Preferably, the host cell is able to convert a carbon source selected from the group consisting of glucose, xylose, arabinose, sucrose, lactose and glycerol. The host cell may for instance be a eukaryotic host cell as described in WO03/062430, WO06/009434, EP1499708B1, WO2006096130 or WO04/099381.

In a further aspect, the present invention relates to a process for the production of a diterpene or diterpene glycoside comprising fermenting a transformed eukaryotic cell according to the present invention in a suitable fermentation medium, and optionally recovering the diterpene and/or diterpene glycoside.

The fermentation medium used in the process for the production of a diterpene or diterpene glycoside may be any suitable fermentation medium which allows growth of a particular eukaryotic host cell. The essential elements of the fermentation medium are known to the person skilled in the art and may be adapted to the host cell selected.

Preferably, the fermentation medium comprises a carbon source selected from the group consisting of plant biomass, celluloses, hemicelluloses, pectines, rhamnose, galactose, fucose, fructose, maltose, maltodextrines, ribose, ribulose, or starch, starch derivatives, sucrose, lactose, fatty acids, triglycerides and glycerol. Preferably, the fermentation medium also comprises a nitrogen source such as ureum, or an ammonium salt such as ammonium sulphate, ammonium chloride, ammoniumnitrate or ammonium phosphate.

The fermentation process according to the present invention may be carried out in batch, fed-batch or continuous mode. A separate hydrolysis and fermentation (SHF) process or a simultaneous saccharification and fermentation (SSF) process may also be applied. A combination of these fermentation process modes may also be possible for optimal productivity. A SSF process may be particularly attractive if starch, cellulose, hemicelluose or pectin is used as a carbon source in the fermentation process, where it may be necessary to add hydrolytic enzymes, such as cellulases, hemicellulases or pectinases to hydrolyse the substrate.

The recombinant microorganism used in the process for the preparation of a diterpene or diterpene glycoside may be any suitable microorganism as defined herein above. It may be advantageous to use a recombinant eukaryotic microorganism according to the invention in the process for the production of a diterpene or diterpene glycoside, because most eukaryotic cells do not require sterile conditions for propagation and are insensitive to bacteriophage infections. In addition, eukaryotic host cells may be grown at low pH to prevent bacterial contamination.

The recombinant microorganism according to the present disclosure may be a facultative anaerobic microorganism. A facultative anaerobic microorganism can be propagated aerobically to a high cell concentration. This anaerobic phase can then be carried out at high cell density which reduces the fermentation volume required substantially, and may minimize the risk of contamination with aerobic microorganisms.

The fermentation process for the production of a diterpene according to the present invention may be an aerobic or an anaerobic fermentation process.

An anaerobic fermentation process may be herein defined as a fermentation process run in the absence of oxygen or in which substantially no oxygen is consumed, preferably less than 5, 2.5 or 1 mmol/L/h, and wherein organic molecules serve as both electron donor and electron acceptors. The fermentation process according to the present invention may also first be run under aerobic conditions and subsequently under anaerobic conditions.

The fermentation process may also be run under oxygen-limited, or micro-aerobical, conditions. Alternatively, the fermentation process may first be run under aerobic conditions and subsequently under oxygen-limited conditions. An oxygen-limited fermentation process is a process in which the oxygen consumption is limited by the oxygen transfer from the gas to the liquid. The degree of oxygen limitation is determined by the amount and composition of the ingoing gasflow as well as the actual mixing/mass transfer properties of the fermentation equipment used.

The production of a diterpene in the process according to the present invention may occur during the growth phase of the host cell, during the stationary (steady state) phase or during both phases. It may be possible to run the fermentation process at different temperatures.

The process for the production of a diterpene or diterpene glycoside may be run at a temperature which is optimal for the eukaryotic cell. The optimum growth temperature may differ for each transformed eukaryotic cell and is known to the person skilled in the art. The optimum temperature might be higher than optimal for wild type organisms to grow the organism efficiently under non-sterile conditions under minimal infection sensitivity and lowest cooling cost. Alternatively, the process may be carried out at a temperature which is not optimal for growth of the recombinant microorganism. Indeed, we have shown that a process for the preparation of a diterpene or diterpene glycoside may be carried out beneficially at a sub-optimal growth temperature of a recombinant microorganism.

The temperature for growth of the recombinant microorganism in a process for production of a diterpene or diterpene glycoside may be above 20°C, 22°C, 25°C, 28°C, or above 30°C, 35°C, or above 37°C, 40°C, 42°C, and preferably below 45°C. During the production phase of a diterpene or diterpene glycoside however, the optimum temperature might be lower than average in order to optimize biomass stability. The temperature during this phase may be below 45°C, for instance below 42°C, 40°C, 37°C, for instance below 35°C, 30°C, or below 28°C, 25°C, 22°C or below 20°C preferably above 15°C.

The invention thus provides a process for the preparation of a diterpene or glycosylated diterpene which process comprises fermenting a recombinant microorganism capable of producing a diterpene or glycosylate diterpene in a suitable fermentation medium at a temperature of about 29°C or less, and optionally recovering the diterpene or glycosylated diterpene. The microorganism may be a microorganism according to the invention.

The temperature of fermentation in such a process may be about 29°C or less, about 28°C or less, about 27°C or less, about 26°C or less or at a lower temperature.

The process for the production of a diterpene or diterpene glycoside according to the present invention may be carried out at any suitable pH value. If the recombinant microorganism is yeast, the pH in the fermentation medium preferably has a value of below 6, preferably below 5,5, preferably below 5, preferably below 4,5, preferably below 4, preferably below pH 3,5 or below pH 3,0, or below pH 2,5, preferably above pH 2. An advantage of carrying out the fermentation at these low pH values is that growth of contaminant bacteria in the fermentation medium may be prevented.

Such a process may be carried out on an industrial scale.

The product of such a process may be one or more of steviolmonoside, steviolbioside, stevioside or rebaudioside A, rebaudioside B, rebaudioside C, rebaudioside D, rebaudioside E, rebaudioside F, rubusoside, dulcoside A. Preferably, rebaudioside A or rebaudioside D is produced.

Recovery of the diterpene or diterpene glycoside from the fermentation medium may be performed by known methods in the art, for instance by distillation, vacuum extraction, solvent extraction, or evaporation.

In the process for the production of a diterpene or diterpene glycoside according to the invention, it may be possible to achieve a concentration of above 5 mg/l fermentation broth, preferably above 10 mg/l, preferably above 20 mg/l, preferably above 30 mg/l fermentation broth, preferably above 40 mg/l, more preferably above 50 mg/l, preferably above 60 mg/l, preferably above 70, preferably above 80 mg/l, preferably above 100 mg/l, preferably above 1 g/l, preferably above 5 g/l, preferably above 10 g/l, but usually below 70 g/l.

The present disclosure also relates to a fermentation broth comprising a diterpene and/or diterpene glycoside obtainable by the process according to the present disclosure. The diterpene or glycosylated diterpene may be a steviol glycoside, in particular rebaudioside A or rebaudioside D.

In the event that a diterpene or diterpene glycoside is expressed within the microorganism, such cells may need to be treated so as to release the diterpene/diterpene glycoside.

The invention also relates to a method for converting a first glycosylated diterpene into a second glycosylated diterpene, which method comprises:
contacting said first glycosylated diterpene with a microorganism according to the invention,
thereby to convert the first glycosylated diterpene into the second glycosylated diterpene.

The second glycosylated diterpene may be rebaudioside A or rebuadioside D. In particular, the method may be carried out in a format such that the first glycosylated diterpene is rebaudioside A and the second glycosylated diterpene is rebaudioside D.

The diterpene or diterpene glycoside, for example rebaudioside A or rebuadioside D, produced by the fermentation process according to the present invention may be used in any application known for such compounds. In particular, they may for instance be used as a sweetener, for example in a food or a beverage. For example steviol glycosides may be formulated in soft drinks, as a tabletop sweetener, chewing gum, dairy product such as yoghurt (eg. plain yoghurt), cake, cereal or cereal-based food, nutraceutical, pharmaceutical, edible gel, confectionery product, cosmetic, toothpastes or other oral cavity composition, etc. In addition, a diterpene or diterpene glycoside can be used as a sweetener not only for drinks, foodstuffs, and other products dedicated for human consumption, but also in animal feed and fodder with improved characteristics.

Accordingly, the disclosure provides, *inter alia,* a foodstuff, feed or beverage which comprises a diterpene or glycosylated prepared according to a process of the disclosure.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

The diterpene or diterpene glycoside obtained in this disclosure can be used in dry or liquid forms. It can be added before or after heat treatment of food products. The amount of the sweetener depends on the purpose of usage. It can be added alone or in the combination with other compounds.

Compounds produced according to the method of the invention may be blended with one or more further non-calorific or calorific sweeteners. Such blending may be used to improve flavour or temporal profile or stability. A wide range of both non-calorific and calorific sweeteners may be suitable for blending with steviol glycosides. For example, non-calorific sweeteners such as mogroside, monatin, aspartame, acesulfame salts, cyclamate, sucralose, saccharin salts or erythritol. Calorific sweeteners suitable for blending with steviol glycosides include sugar alcohols and carbohydrates such as sucrose, glucose, fructose and HFCS. Sweet tasting amino acids such as glycine, alanine or serine may also be used.

The diterpene or diterpene glycoside can be used in the combination with a sweetener suppressor, such as a natural sweetener suppressor. It may be combined with an umami taste enhancer, such as an amino acid or a salt thereof.

A diterpene or diterpene glycoside can be combined with a polyol or sugar alcohol, a carbohydrate, a physiologically active substance or functional ingredient (for example a carotenoid, dietary fiber, fatty acid, saponin, antioxidant, nutraceutical, flavonoid, isothiocyanate, phenol, plant sterol or stanol (phytosterols and phytostanols), a polyols, a prebiotic, a probiotic, a phytoestrogen, soy protein, sulfides/thiols, amino acids, a protein, a vitamin, a mineral, and/or a substance classified based on a health benefits, such as cardiovascular, cholesterol-reducing or anti-inflammatory.

A composition with a diterpene or diterpene glycoside may include a flavoring agent, an aroma component, a nucleotide, an organic acid, an organic acid salt, an inorganic acid, a bitter compound, a protein or protein hydrolyzate, a surfactant, a flavonoid, an astringent compound, a vitamin, a dietary fiber, an antioxidant, a fatty acid and/or a salt.

A diterpene or diterpene glycoside of the disclosure may be applied as a high intensity sweetener to produce zero calorie, reduced calorie or diabetic beverages and food products with improved taste characteristics. Also it can be used in drinks, foodstuffs, pharmaceuticals, and other products in which sugar cannot be used.

In addition, a diterpene or diterpene glycoside of the disclosure may be used as a sweetener not only for drinks, foodstuffs, and other products dedicated for human consumption, but also in animal feed and fodder with improved characteristics.

The examples of products where a diterpene or diterpene glycoside of the disclosure composition can be used as a sweetening compound can be as alcoholic beverages such as vodka, wine, beer, liquor, sake, etc; natural juices, refreshing drinks, carbonated soft drinks, diet drinks, zero calorie drinks, reduced calorie drinks and foods, yogurt drinks, instant juices, instant coffee, powdered types of instant beverages, canned products, syrups, fermented soybean paste, soy sauce, vinegar, dressings, mayonnaise, ketchups, curry, soup, instant bouillon, powdered soy sauce, powdered vinegar, types of biscuits, rice biscuit, crackers, bread, chocolates, caramel, candy, chewing gum, jelly, pudding, preserved fruits and vegetables, fresh cream, jam, marmalade, flower paste, powdered milk, ice cream, sorbet, vegetables and fruits packed in bottles, canned and boiled beans, meat and foods boiled in sweetened sauce, agricultural vegetable food products, seafood, ham, sausage, fish ham, fish sausage, fish paste, deep fried fish products, dried seafood products, frozen food products, preserved seaweed, preserved meat, tobacco, medicinal products, and many others. In principal it can have unlimited applications.

The sweetened composition comprises a beverage, non-limiting examples of which include non-carbonated and carbonated beverages such as colas, ginger ales, root beers, ciders, fruit-flavored soft drinks (e.g., citrus-flavored soft drinks such as lemon-lime or orange), powdered soft drinks, and the like; fruit juices originating in fruits or vegetables, fruit juices including squeezed juices or the like, fruit juices containing fruit particles, fruit beverages, fruit juice beverages, beverages containing fruit juices, beverages with fruit flavorings, vegetable juices, juices containing vegetables, and mixed juices containing fruits and vegetables; sport drinks, energy drinks, near water and the like drinks (e.g., water with natural or synthetic flavorants); tea type or favorite type beverages such as coffee, cocoa, black tea, green tea, oolong tea and the like; beverages containing milk components such as milk beverages, coffee containing milk components, cafe au lait, milk tea, fruit milk beverages, drinkable yogurt, lactic acid bacteria beverages or the like; and dairy products.

Generally, the amount of sweetener present in a sweetened composition varies widely depending on the particular type of sweetened composition and its desired sweetness. Those of ordinary skill in the art can readily discern the appropriate amount of sweetener to put in the sweetened composition.

The diterpene or diterpene glycoside of the disclosure obtained in this disclosure can be used in dry or liquid forms. It can be added before or after heat treatment of food products. The amount of the sweetener depends on the purpose of usage. It can be added alone or in the combination with other compounds.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

Thus, compositions of the present disclosure can be made by any method known to those skilled in the art that provide homogenous even or homogeneous mixtures of the ingredients. These methods include dry blending, spray drying, agglomeration, wet granulation, compaction, co-crystallization and the like.

In solid form a diterpene or diterpene glycoside of the disclosure of the present disclosure can be provided to consumers in any form suitable for delivery into the comestible to be sweetened, including sachets, packets, bulk bags or boxes, cubes, tablets, mists, or dissolvable strips. The composition can be delivered as a unit dose or in bulk form.

For liquid sweetener systems and compositions convenient ranges of fluid, semi-fluid, paste and cream forms, appropriate packing using appropriate packing material in any shape or form shall be invented which is convenient to carry or dispense or store or transport any combination containing any of the above sweetener products or combination of product produced above.

The composition may include various bulking agents, functional ingredients, colorants, flavors.

A reference herein to a patent document or other matter which is given as prior art is not to be taken as an admission that that document or matter was known or that the information it contains was part of the common general knowledge as at the priority date of any of the claims.

The invention comprises the following embodiments:
1. A recombinant microorganism which is a *Yarrowia,* such as *Yarrowia lipolytica,* comprising one or more nucleotide sequence(s) encoding:
   a polypeptide having ent-copalyl pyrophosphate synthase activity;
   a polypeptide having ent-Kaurene synthase activity;
   a polypeptide having ent-Kaurene oxidase activity;
   a polypeptide having kaurenoic acid 13-hydroxylase activity,
   a polypeptide having NADPH-cytochrome p450 reductase activity; and
   a polypeptide having UDP-glucosyltransferase activity,
   wherein a nucleotide sequence encoding a polypeptide having ent-Kaurene oxidase activity encodes a polypeptide comprising an amino acid sequence that has at least 20% sequence identity with the amino acid sequence of SEQ ID NO: 86; and
   wherein a nucleotide sequence encoding a polypeptide having kaurenoic acid 13-hydroxylase activity encodes a polypeptide comprising an amino acid sequence that has at least 20% sequence identity with the amino acid sequence of SEQ ID NO: 34,
   whereby expression of the nucleotide sequence(s) confer(s) on the microorganism the ability to produce at least one of steviolmonoside, steviolbioside, stevioside or rebaudioside A, rebaudioside B, rebaudioside C, rebaudioside D, rebaudioside E, rebaudioside F, rubusoside, dulcoside A.
2. A recombinant microorganism according to embodiment 1, wherein the microorganism comprises a nucleotide sequence encoding a polypeptide capable of catalyzing the addition of a C-13-glucose to steviol,
   whereby expression of the nucleotide sequence confers on the microorganism the ability to produce at least steviolmonoside.
3. A recombinant microorganism according to embodiment 1 or 2, wherein the microorganism comprises a nucleotide sequence encoding a polypeptide capable of catalyzing the addition of a glucose at C-13 position of steviol or steviolmonoside,
   whereby expression of the nucleotide sequence confers on the microorganism the ability to produce at least steviolbioside.
4. A recombinant microorganism according to any one of embodiments 1 to 3, wherein the microorganism comprises a nucleotide sequence encoding a polypeptide capable of catalyzing the addition of a C-19-glucose to steviolbioside,
   whereby expression of the nucleotide sequence confers on the microorganism the ability to produce at least stevioside.
5. A recombinant microorganism according to any one of embodiments 1 to 4, wherein the microorganism comprises a nucleotide sequence encoding a polypeptide capable of catalyzing glucosylation of the C-3' of the glucose at the C-13 position of stevioside,
   whereby expression of the nucleotide sequence confers on the microorganism the ability to produce at least rebaudioside A.
6. A recombinant microorganism according to any one of embodiments 1 to 5, wherein the microorganism comprises a nucleotide sequence encoding a polypeptide capable of catalyzing the glucosylation of stevioside or rebaudioside A,
   whereby expression of the nucleotide sequence confers on the microorganism the ability to produce at least rebaudioside D.
7. A recombinant microorganism according to any one of embodiments 1 to 6, wherein the microorganism comprises a nucleotide sequence encoding a polypeptide capable of catalyzing the glucosylation of stevioside,
   whereby expression of the nucleotide sequence confers on the microorganism the ability to produce at least rebaudioside E.
8. A recombinant microorganism according to any one of embodiments 1 to 7, wherein the microorganism comprises a nucleotide sequence encoding a polypeptide capable of catalyzing the glucosylation of rebaudioside E,
   whereby expression of the nucleotide sequence confers on the microorganism the ability to produce at least rebaudioside D.
9. A recombinant microorganism according to any one of the preceding embodiments, wherein the ability of the microorganism to produce geranylgeranyl diphosphate (GGPP) is upregulated.
10. A recombinant microorganism according to embodiment 9, comprising one or more nucleotide sequence(s) encoding hydroxymethylglutaryl-CoA reductase, farnesyl-pyrophosphate synthetase and geranylgeranyl diphosphate synthase, whereby expression of the nucleotide sequence(s) confer(s) on the microorganism the ability to produce elevated levels of GGPP.
11. A process for the preparation of a diterpene or glycosylated diterpene which comprises fermenting a microorganism according to any one of the preceding embodiments in a suitable fermentation medium, optionally at a temperature of about 29oC or less and optionally recovering the diterpene or glycosylated diterpene.
12. A method for converting a first glycosylated diterpene into a second glycosylated diterpene, which method comprises:
   contacting said first glycosylated diterpene with a microorganism according to any one of embodiments 1 to 10,
   thereby to convert the first glycosylated diterpene into the second glycosylated diterpene.
   The present disclosure is further illustrated by the following Examples:

### EXAMPLES

### General

Standard genetic techniques, such as overexpression of enzymes in the host cells, as well as for additional genetic modification of host cells, are known methods in the art, such as described in Sambrook and Russel (2001) "Molecular Cloning: A Laboratory Manual (3rd edition), Cold Spring Harbor Laboratory, Cold Spring Harbor Laboratory Press*,* or F. Ausubel et al, eds., "Current protocols in molecular biology", Green Publishing and Wiley Interscience, New York (1987). Methods for transformation and genetic modification of fungal host cells are known from e.g. EP-A-0 635 574, WO 98/46772, WO 99/60102 and WO 00/37671.

A description of the sequences is set out in Table 1. Sequences described herein may be defined with reference to the sequence listing or with reference to the database accession numbers also set out in Table 1.

### Example 1: Cloning and expression of the Rebaudioside A biosynthesis route in Saccharomyces cerevisiae (comparative example)

### 1.1. Expression constructs

SEQ ID NO 79, 81 and 83 are obtained by the codon-pair method as disclosed in PCT/EP2007/05594 for *S. cerevisiae* and synthesized at DNA 2.0. Sequence ID nos 79, 81 and 83 are cloned behind constitutive promoters, after the coding sequence a terminator is placed. The expression construct pRS414rebA-01 containing the 3 expression cassettes is created by ligating restriction fragments consisting of synthetic gene constructs into the S. *cerevisiae* expression vector pRS414 (Sirkoski R.S. and Hieter P, Genetics, 1989, 122(1):19-27), based on the multiple cloning site present in this vector. The ligation mix is used for transformation of *E. coli* DH10B (Invitrogen) resulting in the yeast expression construct pGBS414rebA-01.

The sequences of SEQ ID NOs 1, 3, 5, 7, 9, 11, 13, 15, 17, 19, 21, 23, 25, 27, 29, 31, 33, 53, 55, 57, 59, 61, 63, 65, 67, 69 and 77 are obtained by the codon-pair method as disclosed in PCT/EP2007/05594 for *S. cerevisiae* and synthesized at DNA 2.0. Sequence ID no 1, 3, 5, 7, 9, 11, 13, 15, 17, 19, 21, 23, 25, 27, 29, 31, 33, 53, 55, 57, 59, 61, 63, 65, 67, 69 and 77 are cloned behind constitutive promoters, after the coding sequence a terminator is placed. The expression construct library pRS415rebA-01 containing various combinations of expression cassettes containing sequence ID no 1, 3, 5, 7, 9, 11, 13, 15, 17, 19, 21, 23, 25, 27, 29, 31, 33, 53, 55, 57, 59, 61, 63, 65, 67, 69 and 77 is created by ligating restriction fragments consisting of synthetic gene constructs into the *S. cerevisiae* expression vector pRS415 (Sirkoski R.S. and Hieter P, Genetics, 1989, 122(1):19-27), based on the multiple cloning site present in this vector. The ligation mix is used for transformation of *E. coli* DH10B (Invitrogen) resulting in the yeast expression construct library pGBS415rebA-01.

The sequences of SEQ ID NO 35, 37, 39, 41, 43, 45, 47, 49, 51, 71, 73, or 75 are obtained by the codon-pair method as disclosed in PCT/EP2007/05594 for *S. cerevisiae* and synthesized at DNA 2.0. Sequence ID no 35, 37, 39, 41, 43, 45, 47, 49, 51, 71, 73, or 75 are cloned behind constitutive promoters, after the coding sequence a terminator is placed. The expression construct library pRS416rebA-01 containing various combinations of expression cassettes containing seq ID no 35, 37, 39, 41, 43, 45, 47, 49, 51, 71, 73, or 75 is created by ligating restriction fragments consisting of synthetic gene constructs into the S. *cerevisiae* expression vector pRS416 (Sirkoski R.S. and Hieter P, Genetics, 1989, 122(1):19-27), based on the multiple cloning site present in this vector. The ligation mix is used for transformation of *E. coli* DH10B (Invitrogen) resulting in the yeast expression construct library pGBS416rebA-01.

### 1.2 Transformation

The expression plasmid pGBS414rebA-01 and the expression plasmid libraries pGBS415rebA-01 and pGBS416rebA-01 are transformed into *S. cerevisiae* strain CEN.PK113-6B (*MATA ura 3 52 leu 2 112* t*rp1-289*). Transformation mixtures are plated on Yeast Nitrogen Base (YNB) w/o AA (Difco) + 2% glucose.

### 1.3 Cultivation of transformants.

Transformants are inoculated in Verduyn medium comprising glucose, galactose or other C-sources like ethanol or glycerol, supplemented with appropriate amino acids if required (Verduyn et al., 1992, Yeast. Jul;8(7):501-17) and grown under aerobic, anaerobic and oxygen-limited conditions in shake flasks. The medium for anaerobic cultivation is supplemented with 0.01 g/l ergosterol and 0.42 g/l Tween 80 dissolved in ethanol (Andreasen and Stier, 1953, J. cell. Physiol, 41, 23-36; Andreasen and Stier, 1954, J. Cell. Physiol, 43: 271-281). All yeast cultures are grown at 30°C in a shaking incubator at 250-280 rpm.

### 1.4 Detection of rebaudioside A

At different incubation times, aliquots of the cultures are removed, centrifuged and the medium and cells are analysed by LC/MS for formation of rebA, as described below. After growth overnight the cells are spun down and the Rebaudioside A (C44H70O23, Mmonoisotopic = 966.43079) concentration is measured in the by LC/MS as described below. For this purpose an UHPLC system is coupled to a triple quadrupole mass spectrometer and rebaudiosisde A is detected in MS/MS mode by the loss of a hexose unit from the protonated molecule.

A Waters acquity UPLC BEH amide column (1.7 um, 2.1*150 mm) is used with isocratic elution. The mobile phase consists of acetonitrile/10 mM NH4Ac in MilliQ water (pH 6.8), (8:2 v/v), at a flowrate of 300 ul/min. The injection volume was 5 µL, and the column temperature is kept at 30 degrees Celcius.

### Example 2. Over-expression of ERG20, BTS1 and tHMG in S. cerevisiae (comparative example)

For over-expression of ERG20, BTS1 tHMG1, expression cassettes were designed to be integrated in one locus using technology described in co-pending patent application no. US61/616254. To amplify the 5' and 3' integration flanks for the integration locus, suitable primers and genomic DNA from a CEN.PK yeast strain (van Dijken et al. Enzyme and Microbial Technology 26 (2000) 706-714) was used. The different genes were ordered as cassettes (containing homologous sequence, promoter, gene, terminator, homologous sequence) at DNA2.0. The genes in these cassettes were flanked by constitutive promoters and terminators. See Table 2. Plasmid DNA from DNA2.0 containing the ERG20, tHMG1 and BTS1 cassettes were dissolved to a concentration of 100 ng/µl. In a 50 µl PCR mix 20 ng template was used together with 20 pmol of the primers. The material was dissolved to a concentration of 0.5 µg/µl.

**Table 2: Composition of the over-expression constructs.**

| ***Promoter*** | ***ORF*** | ***Terminator*** |
|---|---|---|
| Eno2 (SEQ ID NO: 201) | Erg20 (SEQ ID NO: 81) | Adh1 (SEQ ID NO: 212) |
| Fba1 (SEQ ID NO: 202) | tHMG1 (SEQ ID NO: 79) | Adh2 (SEQ ID NO: 213) |
| Tef1 (SEQ ID NO: 203) | Bts1 (SEQ ID NO:83) | Gmpl (SEQ ID NO: 214) |

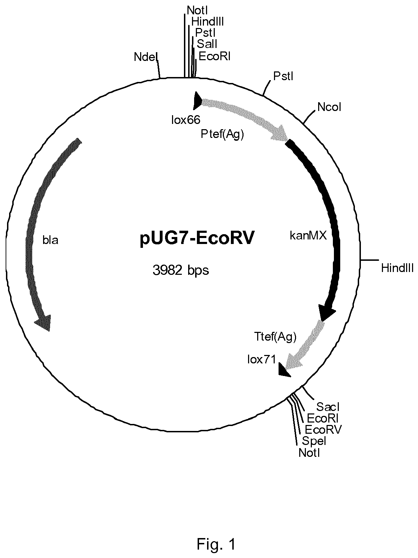

For amplification of the selection marker, the pUG7-EcoRV construct (Figure 1) and suitable primers were used. The KanMX fragment was purified from gel using the Zymoclean Gel DNA Recovery kit (ZymoResearch). Yeast strain Cen.PK113-3C was transformed with the fragments listed in Table 3.

**Table 3: DNA fragments used for transformation of ERG20, tHMG1 and BTS1**

| ***Fragment*** |
|---|
| 5'YPRcTau3 |
| ERG20 cassette |
| tHMG1 cassette |
| KanMX cassatte |
| BTS1 cassette |
| 3'YPRcTau3 |

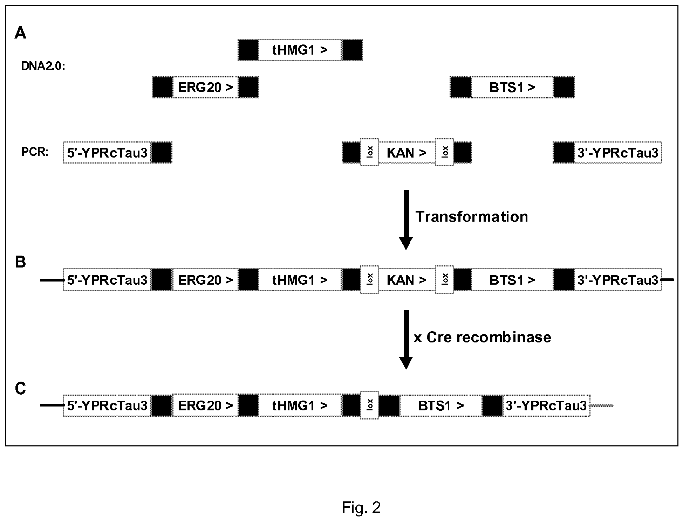

After transformation and recovery for 2.5 hours in YEPhD (yeast extract phytone peptone glucose; BBL Phytone Peptone from BD) at 30°C the cells were plated on YEPhD agar with 200 µg/ml G418 (Sigma). The plates were incubated at 30°C for 4 days. Correct integration was established with diagnostic PCR and sequencing. Over-expression was confirmed with LC/MS on the proteins. The schematic of the assembly of ERG20, tHMG1 and BTS1 is illustrated in Figure 2. This strain is named STV002.

Expression of the CRE-recombinase in this strain led to out-recombination of the KanMX marker. Correct out-recombination, and presence of ERG20, tHMG and BTS1 was established with diagnostic PCR.

### Example 3. Knock down of Erg9 (comparative example)

For reducing the expression of Erg9, an Erg9 knock down construct was designed and used that contains a modified 3' end, that continues into the TRP1 promoter driving TRP1 expression.

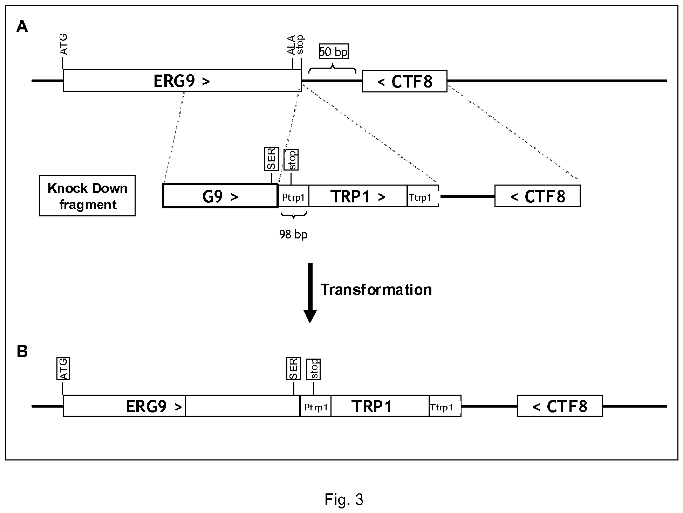

The construct containing the Erg9-KD fragment was transformed to *E. coli* TOP10 cells. Transformants were grown in 2PY(2 times Phytone peptone Yeast extract), sAMP medium. Plasmid DNA was isolated with the QIAprep Spin Miniprep kit (Qiagen) and digested with Sall-HF (New England Biolabs). To concentrate, the DNA was precipitated with ethanol. The fragment was transformed to S. *cerevisiae,* and colonies were plated on mineral medium (Verduyn et al, 1992. Yeast 8:501-517) agar plates without tryptophan. Correct integration of the Erg9-KD construct was confirmed with diagnostic PCR and sequencing. The schematic of performed transformation of the Erg9-KD construct is illustrated in Figure 3. The strain was named STV003.

### Example 4. Over-expression of UGT2 1a (comparative example)

For over-expression of UGT2_1a, technology was used as described in co-pending patent application nos. US61/616254 and EP12159094.7. The UGT2a was ordered as a cassette (containing homologous sequence, promoter, gene, terminator, homologous sequence) at DNA2.0. For details, see Table 4. To obtain the fragments containing the marker and Cre-recombinase, technology was used as described in co-pending patent application no. EP12159094.7. The NAT marker, conferring resistance to nourseothricin was used for selection.

**Table 4: Composition of the over-expression construct**

| ***Promoter*** | ***ORF*** | ***Terminator*** |
|---|---|---|
| Pgk1 (SEQ ID NO: 204) | UGT2_1a (SEQ ID NO: 87) | Adh2 (SEQ ID NO: 213) |

Suitable primers were used for amplification. To amplify the 5' and 3' integration flanks for the integration locus, suitable primers and genomic DNA from a CEN.PK yeast strain was used.

*S. cerevisiae* yeast strain STV003 was transformed with the fragments listed in Table 5, and the transformation mix was plated on YEPhD agar plates containing 50 µg/ml nourseothricin (Lexy NTC from Jena Bioscience).

**Table 5: DNA fragments used for transformation of UGT2a**

| ***Fragment*** |
|---|
| 5'Chr09.01 |
| UGT2a cassette |
| NAT-CR |
| RE |
| 3'Chr09.01 |

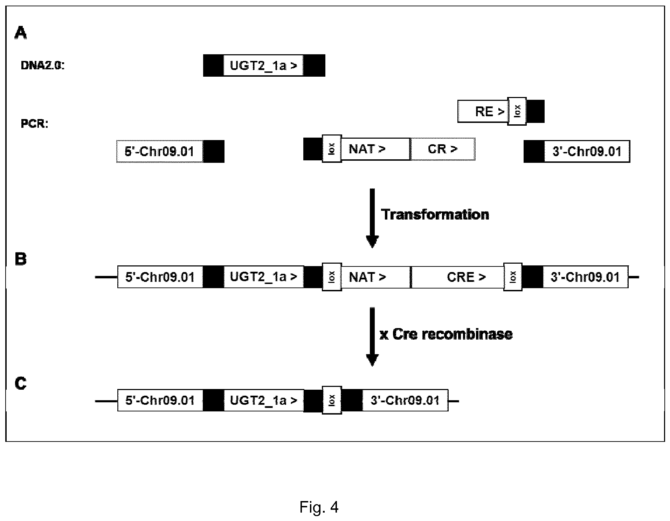

Expression of the CRE recombinase is activated by the presence of galactose. To induce the expression of the CRE recombinase, transformants were restreaked on YEPh Galactose medium. This resulted in out-recombination of the marker(s) located between lox sites. Correct integration of the UGT2a and out-recombination of the NAT marker was confirmed with diagnostic PCR. The resulting strain was named STV004. The schematic of the performed transformation of the UGT2a construct is illustrated in Figure 4.

### Example 5. Over-expression of production pathway to Steviol: CPS, KS, KO, KAH and CPR (comparative example)

### Steviol pathway

All pathway genes leading to the production of Steviol were designed for integration in tandem in one locus using technology described in co-pending patent application no. US61/616254. To amplify the 5' and 3' integration flanks for the integration locus, suitable primers and genomic DNA from a CEN.PK yeast strain was used. The different genes were ordered as cassettes (containing homologous sequence, promoter, gene, terminator, homologous sequence) at DNA2.0 (see Table 6). The DNA from DNA2.0 was dissolved to 100 ng/µl. This stock solution was further diluted to 5 ng/µl, of which 1 µl was used in a 50µl-PCR mixture. The reaction contained 25 pmol of each primer. After amplification, DNA was purified with the NucleoSpin 96 PCR Clean-up kit (Macherey-Nagel) or alternatively concentrated using ethanol precipitation.

**Table 6.**

| ***Promoter*** | ***ORF*** | ***SEQ ID*** | ***Terminator*** |
|---|---|---|---|
| Kl prom 12.pro (SEQ ID NO: 205) | trCPS_SR | 61 | Sc ADH2.ter (SEQ ID NO: 213) |
| Sc PGK1.pro (SEQ ID NO: 204) | trKS_SR | 65 | Sc TAL1.ter (SEQ ID NO: 215) |
| Sc ENO2.pro (SEQ ID NO: 201) | KO_Gibfu | 85 | Sc TPI1.ter (SEQ ID NO: 216) |
| Ag lox_TEF1.pro (SEQ ID NO: 206) | KANMX | 211 | Ag TEF1_lox.ter (SEQ ID NO: 217) |
| Sc TEF1.pro (SEQ ID NO: 203) | KAH_4 | 33 | Sc GPM1.ter (SEQ ID NO: 214) |
| Kl prom 6.pro (SEQ ID NO: 207) | CPR_2 | 55 | Sc PDC1.ter (SEQ ID NO: 218) |

All fragments for the pathway to Steviol, the marker and the flanks (See overview in Table 7) were transformed to *S. cerevisiae* yeast strain STV004. After overnight recovery in YEPhD at 20°C the transformation mixes were plated on YEPhD agar containing 200 µg/ml G418. These were incubated 3 days at 25°C and one night at RT.

**Table 7. DNA fragments used for transformation of CPS, KS, KO, KanMX, KAH and CPR.**

| ***Fragment*** |
|---|
| 5'INT1 |
| CPS cassette |
| KS cassette |
| KO cassette |
| KanMX cassette |
| KAH cassette |
| CPR cassette |
| 3'INT1 |

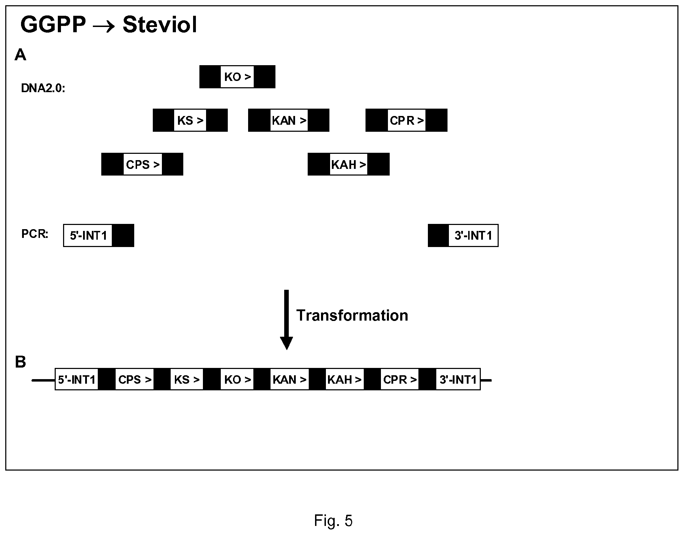

Correct integration was confirmed with diagnostic PCR and sequence analysis (3500 Genetic Analyzer, Applied Biosystems). The sequence reactions were done with the BigDye Terminator v3.1 Cycle Sequencing kit (Life Technologies). Each reaction (10 µl) contained 50 ng template and 3.2 pmol primer. The products were purified by ethanol/EDTA precipitation, dissolved in 10 µl HiDi formamide and applied onto the apparatus. The strain was named STV018. The schematic of how the pathway from GGPP to steviol is integrated into the genome is illustrated in Figure 5.

### Example 6. Over-expression of production pathway to RebA: CPS, KS, KO, KAH, CPR, UGT1, UGT3 and UGT4 (comparative example).

All pathway genes leading to the production of RebA were designed to be integrated in one locus using technology described in co-pending patent application no. US61/616254. To amplify the 5' and 3' integration flanks for the integration locus, suitable primers and genomic DNA from a CEN.PK yeast strain was used. The different genes were ordered as cassettes (containing homologous sequence, promoter, gene, terminator, homologous sequence) at DNA2.0 (see Table 8 for overview). The DNA from DNA2.0 was dissolved to 100 ng/µl. This stock solution was further diluted to 5 ng/µl, of which 1 µl was used in a 50µl-PCR mixture. The reaction contained 25 pmol of each primer. After amplification, DNA was purified with the NucleoSpin 96 PCR Clean-up kit (Macherey-Nagel) or alternatively concentrated using ethanol precipitation.

**Table 8. Sequences used for production pathway to RebA**

| ***Promoter*** | ***ORF*** | ***SEQ ID*** | ***Terminator*** |
|---|---|---|---|
| Kl prom 12.pro (SEQ ID NO: 205) | trCPS_SR | 61 | Sc ADH2.ter(SEQ ID NO: ) |
| Sc PGK1.pro (SEQ ID NO: 204) | trKS_SR | 65 | Sc TAL1.ter (SEQ ID NO: 215) |
| Sc ENO2.pro (SEQ ID NO: 201) | KO_Gibfu | 85 | Sc TPI1.ter (SEQ ID NO: 216) |
| Ag lox-TEF1.pro (SEQ ID NO:206 ) | KANMX | 211 | Ag TEF1_lox.ter (SEQ ID NO: 217) |
| Sc TEF1.pro (SEQ ID NO: 203) | KAH_4 | 33 | Sc GPM1.ter (SEQ ID NO: 214) |
| Kl prom 6.pro (SEQ ID NO: 207) | CPR_3 | 57 | Sc PDC1.ter (SEQ ID NO: 218) |
| Kl prom 3.pro (SEQ ID NO: 221) | UGT1_SR | 71 | Sc TDH1.ter (SEQ ID NO: 219) |
| Kl prom 2.pro (SEQ ID NO: 222) | UGT3_SR | 73 | Sc ADH1.ter (SEQ ID NO: 212) |
| Sc FBA1.pro (SEQ ID NO: 202) | UGT4_SR | 75 | Sc ENO1.ter (SEQ ID NO: 220) |

All fragments for the pathway to RebA, the marker and the flanks (see overview in Table 9) were transformed to *S. cerevisiae* yeast strain STV004. After overnight recovery in YEPhD at 20°C the transformation mixes were plated on YEPhD agar containing 200 µg/ml G418. These were incubated 3 days at 25°C and one night at RT.

**Table 9. DNA fragments used for transformation of CPS, KS, KO, KanMX, KAH, CPR, UGT1, UGT3 and UGT4.**

| ***Fragment*** |
|---|
| 5'INT1 |
| CPS cassette |
| KS cassette |
| KO cassette |
| KanMX cassette |
| KAH cassette |
| CPR cassette |
| UGT1 cassette |
| UGT3 cassette |
| UGT4 cassette |
| 3'INT1 |

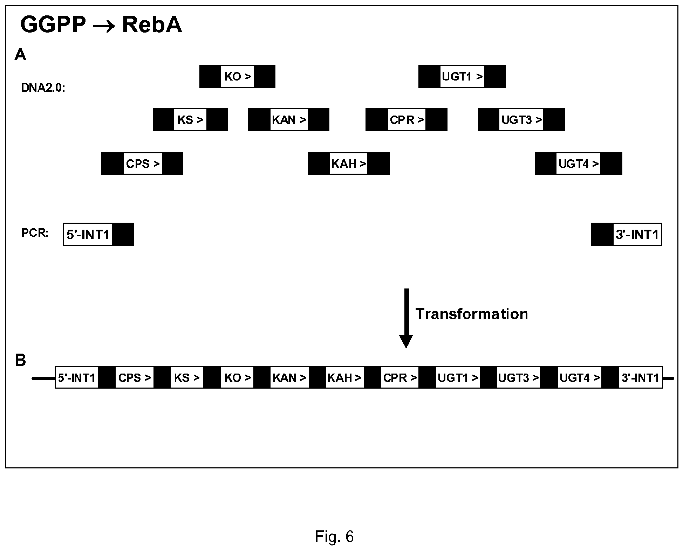

Correct integration was confirmed with diagnostic PCR and sequence analysis (3500 Genetic Analyzer, Applied Biosystems). The sequence reactions were done with the BigDye Terminator v3.1 Cycle Sequencing kit (Life Technologies). Each reaction (10 µl) contained 50 ng template and 3.2 pmol primer. The products were purified by ethanol/EDTA precipitation, dissolved in 10 µl HiDi formamide and applied onto the apparatus. The strain was named STV006. The schematic of how the pathway from GGPP to RebA is integrated into the genome is illustrated in Figure 6.

### Example 7. Steviol production in strain STV018 (comparative example)

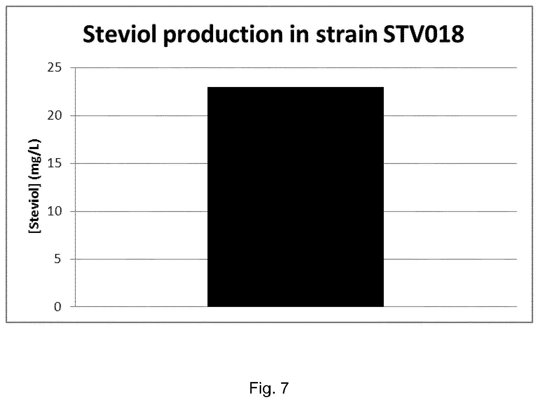

A *S. cerevisiae* yeast strain containing the modifications described in Examples 1,2,3,4 and 5, strain STV018, was grown in YEP (Yeast Extract Peptone) with gluocse. Shake flasks were incubated at 30 degrees C, and incubated 7 days. To extract product from the cells, whole broth was incubated at 95°C for 15 minutes, acetonitrile was added (halve volume equivalent), samples were mixed and spun down. Samples were diluted with 33% acetonitrile when appropriate. Samples were analyzed for Steviol using LC/MS. Steviol (Sigma H8664) was used as standard. Results are illlustrated in Figure 7.

### Example 8. RebA production in strain STV006 (comparative example)

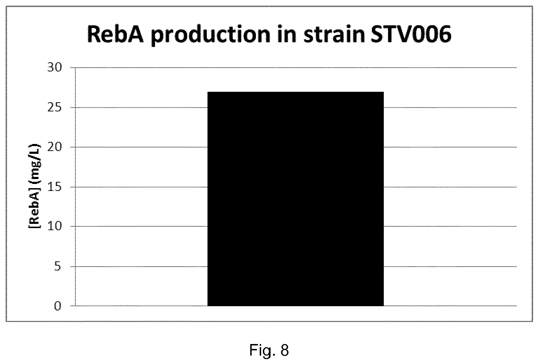

A *S. cerevisiae* yeast strain containing the modifications described in examples 1,2,3,4 and 6, strain STV006, was grown in YEP (Yeast Extract Peptone) with glucose. Shake flasks were incubated at 30 degrees C, and incubated 7 days. To extract product from the cells, whole broth was incubated at 95°C for 15 minutes, acetonitrile was added (halve volume equivalent), samples were mixed and spun down. Samples were diluted with 33% acetonitrile when appropriate. Samples were analyzed for RebA using LC/MS. RebA (RV0141-94, DAE Pyung Co. Ltd) was used as standard. Results are illustrated in Figure 8. Table 10 sets out the strains used in Examples 2 to 8.

**Table 10. Table of strains**

| ***Strain*** | ***Background*** | ***Genotype*** |
|---|---|---|
| Cen.PK113-3C | - | MATa *URA3 HIS3 LEU2 trp1-289 MAL2-8C SUC2* |
| STV002 | Cen.PK113-3C | MATa *URA3 HIS3 LEU2 trp1-289 MAL2-8C SUC2* YPRcTau3::ERG20, tHMG1, KanMX, BTS1 |
| STV003 | STV002 | MATa *URA3 HIS3 LEU2 trp1-289 MAL2-8C SUC2* YPRcTau3::ERG20, tHMG1, KanMX, BTS1 ERG9::ERG9-KD TRP1 |
| STV004 | STV003 | MATa *URA3 HIS3 LEU2 trp1-289 MAL2-8C SUC2* YPRcTau3::ERG20, tHMG1, BTS1 ERG9::ERG9-KD TRP1 Chr09.01::UGT91D2 |
| STV018 | STV004 | MATa *URA3 HIS3 LEU2 trp1-289 MAL2-8C SUC2* YPRcTau3::ERG20, tHMG1, BTS1 ERG9::ERG9-KD TRP1 Chr09.01::UGT91D2 INT1::CPS, KS, KO, KanMX, KAH, CPR |
| STV006 | STV004 | MATa *URA3 HIS3 LEU2 trp1-289 MAL2-8C SUC2* YPRcTau3::ERG20, tHMG1, BTS1 ERG9::ERG9-KD TRP1 Chr09.01::UGT91D2 INT1::CPS, KS, KO, KanMX, KAH, CPR, UGT1, UGT3, UGT4 |

### Example 9. Effect of KO and CPR enzyme variants (comparative example)

We evaluated the effect of different combinations of the KO enzyme and the CPR enzyme on RebA production. These strains were made as described in Example 6, using the same CPS, KS, KAH, UGT1, UGT3 and UGT4 cassettes, but different variants of the KO genes and/or the CPR genes. The KO genes are under the control of the same Sc ENO2.pro promoter and Sc TPI1.ter terminator as described in Example 6. The CPR genes are under the control of the same Kl prom 6.pro promoter and Sc PDC1.ter terminator as described in Example 6.

**Table 11. Strains with different combinations of the KO and CPR enzymes.**

| **Strain** | **KO** | **CPR** |
|---|---|---|
| STV006 | KO_Gibfu (SEQ ID NO: 85) | CPR_3 (SEQ ID NO: 57) |
| STV012 | KO_2 (SEQ ID NO: 23) | CPR_3 (SEQ ID NO: 57) |
| STV016 | KO_2 (SEQ ID NO: 23) | CPR_SR (SEQ ID NO: 59) |
| STV017 | KO_Gibfu (SEQ ID NO: 85) | CPR_SR (SEQ ID NO: 59) |

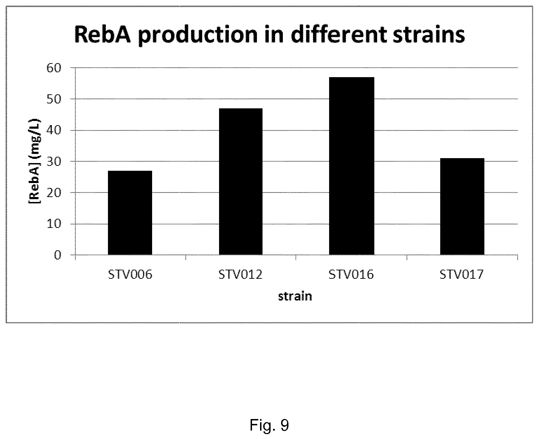

Strains were inoculated in shake flasks and incubated at 30 degrees C for 7 days. To extract product from the cells, whole broth was incubated at 95°C for 15 minutes, acetonitrile was added (halve volume equivalent), samples were mixed and spun down. Samples were diluted with 33% acetonitrile when appropriate. Samples were analyzed for RebA using LC/MS. RebA (RV0141-94, DAE Pyung Co. Ltd) was used as standard. Results are illustrated in Figure 9.

### Example 10. Over-expression of partial production pathway: CPS, KS, KO, KAH, CPR, UGT1 and UGT2 (comparative example)

Using a similar strategy as detailed in Example 6, we constructed a strain expressing CPS, KS, KO, KAH, CPR, UGT1 and UGT2. The same genes are expressed as described for strain STV006 in example 6, except this strain, named STV019, does not contain a UGT3 and UGT4.

### Example 11. Over-expression of partial production pathway: CPS. KS. KO. KAH, CPR, UGT1, UGT2 and UGT4 (comparative example)

Using a similar strategy as detailed in Example 6, we constructed a strain expressing CPS, KS, KO, KAH, CPR, UGT1, UGT2 and UGT4. The genes are expressed as described for strain STV006 in Example 6, except this strain, named STV020, does not contain a UGT4.

### Example 12. Steviol glycoside formation in strains STV018, STV019 and STV020 (comparative example)

*S. cerevisiae* yeast strains containing the modifications described in examples 1, 2, 3, 4, 5, 6, 10 and 11, strains STV018, STV019 and STV020, were grown in YEP (Yeast Extract Peptone) with glucose. Shake flasks were incubated at 30 degrees C, and incubated 7 days. To extract product from the cells, whole broth was treated with heat and acetonitrile, spun down, and the supernatant analyzed for Stevioside and RebA using LC/MS. RebA (RV0141-94, DAE Pyung Co. Ltd) and stevioside (RV0141-95, DAE Pyung Co. Ltd) were used as standards.

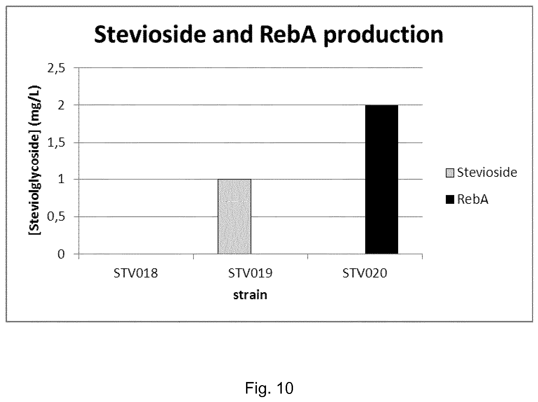

Surprisingly, we found the production of stevioside in strain STV019, and RebA in strain STV020. Results are illustrated in Figure 10.

These results illustrate the activity for the formation of stevioside, without the expression of an UGT3.

### Example 13. Variable KO SR expression (comparative example)

We constructed strains expressing CPS, KS, KO, KAH, CPR, UGT1, UGT2, UGT3 and UGT4 by transformation as described in Examples 1,2,3,4 and 6. As ent-Kaurene oxidase, we used either the KO_SR (SEQ ID NO: 67) or the KO_Gibfu (SEQ ID NO: 85). To drive the expression of ent-Kaurene oxidase, we used promoters of different strength (see Table 12). Production of RebA was determined as described in Example 8. With the two different ent-Kaurene oxidases, opposite effects were observed for the promoter strength.

**Table 12: KO and promoter sequences used in various strains**

| strain | Type of KO | Promoter | Promoter description | RebA (mg/L) |
|---|---|---|---|---|
| STV022 | KO_SR (SEQ ID NO: 67) | PSc_PRE3 (SEQ ID NO: 223) | weak | 50 |
| STV023 | KO_SR (SEQ ID NO: 67) | PSc_ENO2 (SEQ ID NO: 201) | strong | 16 |
| STV024 | KO_Gibfu (SEQ ID NO: 85) | PSc_PRE3 (SEQ ID NO: 223) | weak | 3 |
| STV006 | KO_Gibfu (SEQ ID NO: 85) | PSc_ENO2 (SEQ ID NO: 201) | strong | 27 |

### Example 14. Production of steviol in recombinant Yarrowia lipolytica

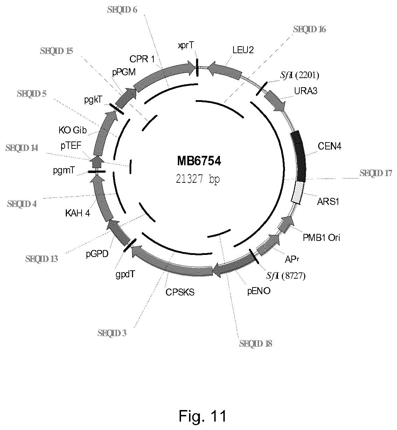

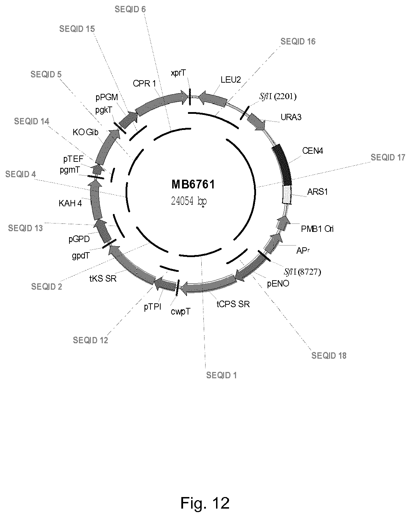

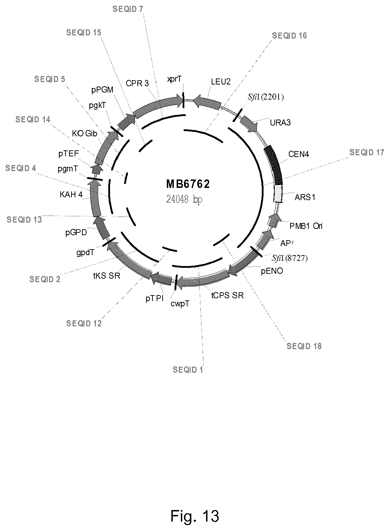

Plasmids pMB6754, pMB6761, and pMB6762 (see Table 13 and Figures 11, 12 and 13) encoding genes for the synthesis of steviol were constructed as follows. Open reading frames for tCPS (SEQ ID NO: 182), tKS (SEQ ID NO: 183), CPSKS (SEQ ID NO: 184), KOGib (SEQ ID NO: 186), KAH4 (SEQ ID NO: 185), CPR1 (SEQ ID NO: 187) and CPR3 (SEQ ID NO: 188) were codon pair optimized using codon pair optimisation technology as disclosed in PCT/EP2007/05594, for expression in *Yarrowia lipolytica.* The optimized sequences, flanked by 60 bp of the desired promoter and terminator, were synthesized by GenScript (SEQ ID NOS: 182-188), and amplified by PCR using appropriate primers. DNA fragments encoding terminator-promoter sequences, TPI promoter, or *Yarrowia lipolytica* markers were amplified by PCR from existing constructs (SEQID 193-197 and 199). Vector DNA (SEQID 198), consisting of the *S. cerevisiae* centromere-based *URA3* plasmid YCp50 (Rose et al., Gene 1987; 60(2-3):237-43) with *ENOp* from *Yarrowia* replacing the *tet* gene using standard techniques, was prepared from *E. coli and* digested with *Xba*I and *Sna*BI. All fragments were purified by gel electrophoresis using a QiaQuick kit (Qiagen). *S. cerevisiae* strain 10556-23C (W303 background; G.R. Fink) was transformed (Gietz and Schiestl, Nat. Protoc. 2007; 2(1): 31-4) with 250 ng of each DNA fragment and selected for prototrophy on minimal glucose aspartate medium. Plasmids were rescued from prototrophic transformants (Nucleic Acids Research, Vol. 20, No. 14, p. 3790 (1992)) and used to transform *E. coli* DH5α to ampicillin resistance (100 mg/L) on LB agar plates.

*Yarrowia* strain ML2597 with increased expression of geranylgeranyldiphosphate synthase was obtained by transformation of MF350 with pMB4591 (*tef1-GGS URA2*) (US7851199). Plasmids pMB6754, pMB6761, and pMB6762 were digested with *Sfi*I and used to transform ML2597 to leucine prototrophy on minimal glucose aspartate medium containing adenine (0.2 mM). Transformants were restreaked to selective medium and subsequently inoculated to 0.8 ml YPD in 24 well microtiter plates (MTP). Plates were sealed with a BugStopper mat (Whatman) and strains were grown for steviol production at 30°C with shaking at 800 rpm for six days in a Multitron incubator (Infors).

500 µl culture broth was transferred to a microfuge tube and 250 µl acetonitrile and 600 µl silica beads (0.5 mm) were added. Cells were disrupted by bead beating for 10 min, and cell debris was pelleted at 13,000xg for 1 min in a microcentrifuge. Supernatant was diluted 200 fold with 1:2 (v:v) acetonitrile:water and analyzed by LC-MS (Table 14). A mixture of steviosides containing 100 µg/ml steviol was used as a standard.

**Table 13. Steviol & RebA pathway plasmids**

| Plasmid | SEQIDs | Genotype (partial) |
|---|---|---|
| pMB6754 | 184,185,186,187,194,195,196,197,198,199 | CPSKS, KAH_4, KO_Gib, CPR_1, LEU2 |
| pMB6761 | 184,185,186,188,194,195,196,197,198,199 | tCPS, tKS, KAH_4, KO_Gib, CPR_1, LEU2 |
| pMB6762 | 182,183,185,186,188,193,194,195,196,197,198,199 | tCPS, tKS, KAH_4, KO_Gib, CPR_3, LEU2 |
| pMB6775 | 189,190,191,192,194,195,196,198,199,200 | UGT1, UGT3, UGT4, UGT2, HPH |

**Table 14. Steviol production**

| Strain | Plasmid | Steviol (mg/l) |
|---|---|---|
| ML12925 | pMB6754 | 5.4 |
| ML12927 | pMB6754 | 6.6 |
| ML12929 | pMB6162 | 6.2 |
| ML12930 | pMB6162 | 5.7 |
| ML12931 | pMB6761 | 7.1 |
| ML12932 | pMB6761 | 7.6 |

### Example 15: production of RebA in recombinant Yarrowia lipolytica

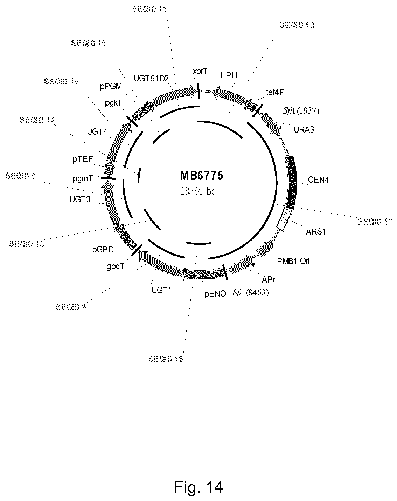

Plasmid pMB6775 (see Table 13 and Figure 14) encoding genes for the synthesis of RebA was constructed as follows. Open reading frames for a UGT1, UGT3, UGT4, and UGT2 were codon pair optimized using codon pair optimisation technology as disclosed in PCT/EP2007/05594, for expression in *Yarrowia lipolytica.* The optimized sequences, flanked by 60 bp of the desired promoter and terminator, were synthesized by GenScript (SEQ ID NOs 189-192), and amplified by PCR using appropriate primers. DNA fragments encoding terminator-promoter sequences or *Yarrowia lipolytica* markers were amplified by PCR from existing constructs (SEQ ID NOS: 194-196,199 and 200). The vector (SEQ ID NO: 198), consisting of the *S. cerevisiae* centromere-based *URA3* plasmid YCp50 (Rose et al., *supra*) with *ENOp* from *Yarrowia* replacing the *tet* gene using standard techniques, was prepared from *E. coli* and digested with *Xba*I and *Sna*BI. All fragments were purified by gel electrophoresis using a QiaQuick kit (Qiagen). *S. cerevisiae* strain 10556-23C (W303 background; G.R. Fink) was transformed (Gietz and Schistl, *supra*) with 250 ng of each DNA fragment and selected for prototrophy on minimal glucose aspartate medium. Plasmids were rescued from prototrophic transformants (Nucleic Acids Research, Vol. 20, No. 14, p. 3790 (1992)) and used to transform *E. coli* DH5α to ampicillin resistance (100 mg/L) on LB agar plates.

Plasmid MB6775 was digested with *Sfi*I and used to transform Steviol producing *Yarrowia* strains ML12925, ML12929, and ML12931 to hygromycin resistance (100 mg/L) on YPD agar plates. Transformants were restreaked to selective medium and subsequently inoculated to 0.8 ml YPD in 24 well microtiter plates (MTP). Plates were sealed with a BugStopper mat (Whatman) and strains are grown for steviol production at 30°C with shaking at 800 rpm for six days in a Multitron incubator (Infors).

Samples consisting of 100 µl culture broth were transferred to a microfuge tube and 700 µl 33% acetonitrile and 600 µl silica beads (0.5 mm) were added. Cells were disrupted by bead beating for 10 min, and cell debris was pelleted at 13,000xg for 1 min in a microcentrifuge. Supernatant was diluted 2.5 fold with 1:2 (v:v) acetonitrile:water and analyzed by LC-MS (Table 15). Additionally, 500 µl culture broth was pelleted, and supernatant was diluted 20 fold prior to LC-MS analysis (Table Z). RebA was used as a standard.

**Table 15. RebA production**

| Parent Strain | Transformant | RebA (mg/l) | |
|---|---|---|---|
| | | whole broth | supernatant |
| ML12925 | 1 | 1.7 | 1.1 |
| ML12925 | 2 | 1.6 | 1.0 |
| ML12925 | 3 | 1.4 | 1.1 |
| ML12925 | 4 | 1.2 | 0.8 |
| ML12929 | 1 | 4.2 | 3.9 |
| ML12929 | 2 | 3.6 | 3.6 |
| ML12929 | 3 | 3.4 | 3.4 |
| ML12929 | 4 | 3.3 | 3.0 |
| ML12931 | 1 | 1.4 | 1.0 |
| ML12931 | 2 | 1.4 | 1.0 |
| ML12931 | 3 | 1.3 | 0.8 |
| ML12931 | 4 | 1.2 | 1.1 |

### Example 16: Rebaudioside D (RebD) biosynthesis in yeast (comparative example)

Variation in the expression level of the genes of a metabolic pathway may result in variation in product and by-product formation. Changing the expression levels of genes who's gene products compete for the same substrate may alter the amount and nature of the products produced. One approach to change the expression level of a gene, is by changing the strength of the promoter driving the expression of that gene. Different conditions may also alter the expression of genes. We made use of different promoters to alter the expression of the UGTs described in Example 6.

All pathway genes leading to the production of RebD were designed to be integrated in one locus in the STV004 strain background. To amplify the 5' and 3' integration flanks for the integration locus (site 3), suitable primers and genomic DNA from a CEN.PK yeast strain was used. The different genes were ordered as cassettes (containing homologous sequence, promoter, gene, terminator, homologous sequence) at DNA2.0 (see Table 16 for overview). The DNA from DNA2.0 was dissolved to 100 ng/µl. This stock solution was further diluted to 5 ng/µl, of which 1 µl was used in a 50µl-PCR mixture. The reaction contained 25 pmol of each primer. After amplification, DNA was purified with the NucleoSpin 96 PCR Clean-up kit (Macherey-Nagel) or alternatively concentrated using ethanol precipitation.

**Table 16. Composition of the over-expression constructs for CPS, KS, KO, KAH, CPR, UGT1, UGT3 and UGT4.**

| ***Promoter*** | ***ORF*** | ***Terminator*** |
|---|---|---|
| KI prom 12.pro (SEQ ID NO: 205) | CPS (SEQ ID NO: 61) | Sc Adh2.ter (SEQ ID NO: 213) |
| Sc Pgk1.pro (SEQ ID NO: 204) | KS (SEQ ID NO: 65) | Sc Tall.ter (SEQ ID NO: 215) |
| Sc Eno2.pro (SEQ ID NO: 201) | KO (SEQ ID NO: 85) | Sc Tpil.ter (SEQ ID NO: 216) |
| Ag lox_Tef1.pro (SEQ ID NO: 206) | KANMX (SEQ ID NO: 211) | Ag Tef1_lox.ter (SEQ ID NO:217) |
| Sc Tef1.pro (SEQ ID NO: 203) | KAH (SEQ ID NO: 33) | Sc Gpml.ter (SEQ ID NO: 214) |
| KI prom 6.pro (SEQ ID NO: 207) | CPR (SEQ ID NO: 57) | Sc Pdcl.ter (SEQ ID NO: 218) |
| Sc Pma1.pro (SEQ ID NO: 208) | UGT1 (SEQ ID NO: 71) | Sc Tdhl.ter (SEQ ID NO: 219) |
| Sc Vps68.pro (SEQ ID NO: 209) | UGT3 (SEQ ID NO: 73) | Sc Adhl.ter (SEQ ID NO: 212) |
| Sc Oye2.pro (SEQ ID NO: 210) | UGT4 (SEQ ID NO: 75) | Sc Enol.ter (SEQ ID NO: 220) |

All fragments for the pathway to RebD, the marker and the flanks (see overview in Table 17) were transformed to a *S. cerevisiae* yeast strain STV004. After overnight recovery in YEPhD at 20°C the transformation mixes were plated on YEPhD agar containing 200 µg/ml G418. These were incubated 3 days at 30°C.

**Table 17. DNA fragments used for transformation of CPS, KS, KO, KanMX, KAH, CPR, UGT1, UGT3 and UGT4.**

| ***Fragment*** |
|---|
| 5' site 3 |
| CPS cassette |
| KS cassette |
| KO cassette |
| KanMX cassette |
| KAH cassette |
| CPR cassette |
| UGT1 cassette |
| UGT3 cassette |
| UGT4 cassette |
| 3'site 3 |

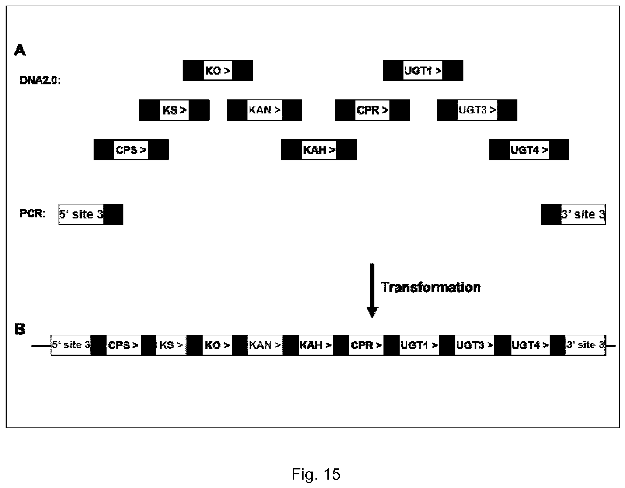

Correct integration was confirmed with diagnostic PCR The resulting strain was named STV015 (MATa *URA3 HIS3 LEU2 trp1-289 MAL2-8C SUC2* site 1::ERG20, tHMG1, BTS1 ERG9::ERG9-KD TRP1 site 2::UGT2 site 3::CPS, KS, KO, KanMX, KAH, CPR, UGT1, UGT3, UGT4). The schematic of the performed transformation of the production pathway is illustrated in Figure 15.

### Example 17. RebD production in strain STV015 (comparative example)

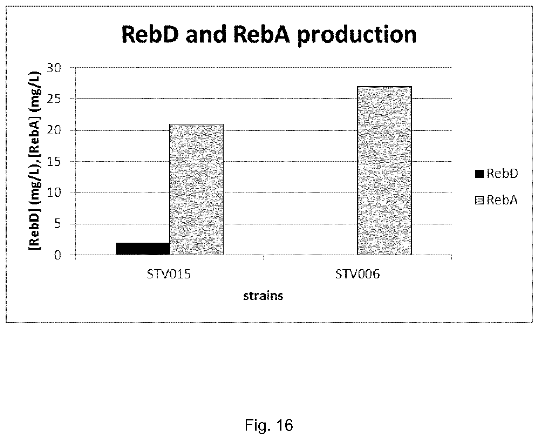

*S. cerevisiae* yeast strain containing the modifications described in examples 1,2,3,4, 6, and 16, strain STV015, was grown in YEP (Yeast Extract Peptone) with glucose. Shake flasks were incubated at 30 degrees C, and incubated 7 days. To extract product from the cells, whole broth was incubated at 95°C for 15 minutes, acetonitrile was added (halve volume equivalent), samples were mixed and spun down. Samples were diluted with 33% acetonitrile when appropriate. Samples were analyzed for RebA and RebD using LC/MS. RebA (RV0141-94, DAE Pyung Co. Ltd) and RebD (ASB-00018229-010, ChromaDex) were used as standards. Results are illustrated in Figure 16.

### Example 18. Enzymatic conversion of diterpenes into glycosylated diterpenes (comparative example)

### Strain

*Saccharomyces cerevisiae* strain STV006 (see Example 6 above) was cultivated on suitable media enabling active transcription and translation of the introduced genes. Obtained cells were pelleted and stored at -20 until analysis.

### Preparation Cell Free Extract

To 8 gr cell pellet 40mL of 100mM Tris buffer pH 7.18 was added and homogenized. Subsequently 8 gr glass beads (50-200um) were added. The samples were cooled on ice for 15 minutes. Cells were disrupted by 4 cycles of each 2 min vortexing on full speed followed by 5 min cooling on ice. After lysis the extract was centrifuged at 3000g for 60 min at 4 degrees C. The obtained supernatant was used directly for activity assays.

### Preparation Permeabilized Cells

8 gr of fresh pellet was homogenized with 40mL of 40% DMSO in Tris buffer (100mM pH 7.18) and frozen at -20 degrees C. Before analysis cells were thawed and 5mL was transferred to a new tube and washed three times with 100mM Tris buffer pH 7.18 containing 0.1% glucose. Cells were spun each time via centrifugation at 3000g. Finally, the cells were resuspended in 5mL of of 100mM Tris pH 7.18.

### UDP-Glucosyltransferases enzyme assay

2.5uL of enzyme sample (CFE, permeabilized cells, isolated or immobilized enzyme) was added to the reaction mixture containing:
- Glucose 0.05% (W/V; 5.55mM)
- UDP-Glc, 5mM
- DMSO, 2.5%(V/V)
Several compounds were added as variables:
- Alkaline phosphatase
- Lactalbumin 10ug/mL
- Metalions: Mn2+, Mg2+, Co2+, Cu2+, Zn2+

### -Steviol intermediate substrates (all dissolved in DSMO).

The total reaction volume was 100uL and the assay were performed in microtiterplates (MTP) heated to 30 degrees C in a MTP eppendorf incubator. Incubations were done while shaking at 800 rpm up to 12 hrs. The MTP's were sealed to prevent contamination and evaporation.

### Analysis

Reaction mixtures were centrifuged at 4 degrees C for 20 minutes to stop the enzyme reaction and collect the samples. 50uL of acetonitrile was added to 100uL of sample in order to completely stop the reaction and extract all molecules formed. To this end the MTP's were sealed and shaken vigorously. Subsequently, the samples were centrifuged for 60 minutes at 4 degrees C and 100ul was transferred to a new MTP for LC-MS analysis.

### Incubations with Steviol

Cell free extracts prepared as described and incubated with either at 0, 30, 300, 3000 uM of steviol. Reactions were performed for 12 hours and analysed by LC-MS.

**Table 18. Steviol glycosides (in ug/ml) using steviol as substrate with Mn2+ as metal ion.**

| uM substrate | Steviol | Stevioside | RebaudiosideB | RebaudiosideA | RebaudiosideD |
|---|---|---|---|---|---|
| 0 | 0 | <0,5 | 5 | 34 | <0,5 |
| 30 | 0 | 4 | 5 | 55 | 0,6 |
| 300 | 0 | 8 | 3 | 60 | 0 |
| 3000 | 209 | 5 | 0 | 67 | 0 |

**Table 19. Effect of metal ions on steviol glycosylation. Steviol glycosides (in ug/ml) using steviol as substrate at 300 uM**

| Metal | Steviol | Stevioside | RebaudiosideB | RebaudiosideA | RebaudiosideD |
|---|---|---|---|---|---|
| Mn2+ | 0 | 8 | 2 | 60 | 0 |
| Mg2+ | 0 | 2 | 13 | 39 | 0 |
| None | 0 | 1 | 21 | 35 | 0 |

### Incubations with Steviobioside

Cell free extracts prepared as described and incubated with either at 0, 30, 300, 3000 uM of Stevioside (RV0141-95, DAE Pyung Co. Ltd). Reactions were performed for 12 hours and analysed by LC-MS.

**Table 20. Steviol glycosides (in ug/ml) using stevioside as substrate with Mn2+ as metal ion.**

| uM substrate | Steviol | Stevioside | RebaudiosideB | RebaudiosideA | RebaudiosideD |
|---|---|---|---|---|---|
| 0 | 0 | <0,5 | 5 | 34 | <0,5 |
| 30 | 0 | 0 | 5 | 77 | 0,7 |
| 300 | 0 | 0 | 15 | 264 | 0 |
| 3000 | 0 | 0 | 16 | 1976 | 0 |

**Table 21. Effect of metalions on stevioside glycosylation. Steviol glycosides (in ug/ml) using stevioside as substrate at 300 uM**

| Metal | Steviol | Stevioside | RebaudiosideB | RebaudiosideA | RebaudiosideD |
|---|---|---|---|---|---|
| Mn2+ | 0 | 0 | 15 | 264 | 0 |
| Mg2+ | 0 | 0 | 65 | 179 | 0 |
| None | 0 | 0 | 86 | 176 | 0 |

### Incubations with RebaudiosideA

Cell free extracts prepared as described and incubated with either at 0, 30, 300, 3000 uM of Rebaudisode (RV0141-94, DAE Pyung Co. Ltd. Purity NMR = 83.4%). Reactions were performed for 12 hours and analysed by LC-MS.

**Table 22. Steviol glycosides (in ug/ml) using RebaudiosideA as substrate with Mn2+ as metal ion.**

| uM substrate | Steviol | Stevioside | RebaudiosideB | RebaudiosideA | RebaudiosideD |
|---|---|---|---|---|---|
| 0 | 0 | <0,5 | 5 | 34 | <0,5 |
| 30 | 0 | 0 | 4 | 76 | 0 |
| 300 | 0 | 0 | 11 | 213 | 1,9 |
| 3000 | 0 | 0 | 0 | 1855 | 0 |

**Table 23. Effect of metal ions on Rebaudioside glycosylation. Steviol glycosides (in ug/ml) using RebaudiosideA as substrate at 300 uM**

| Metal | Steviol | Stevioside | RebaudiosideB | RebaudiosideA | RebaudiosideD |
|---|---|---|---|---|---|
| Mn2+ | 0 | 0 | 11 | 213 | 1,9 |
| Mg2+ | 0 | 1,6 | 93 | 163 | 1,4 |
| None | 0 | 0 | 103 | 155 | 1,3 |

### Incubations with RebaudiosideA

Permeabilized cells prepared as described and incubated with either at 0, 30, 300, 3000 uM of Rebaudisode (RV0141-94, DAE Pyung Co. Ltd. Purity NMR = 83.4%). Reactions were performed for 12 hours and analysed by LC-MS.

**Table 24. Steviol glycosides (in ug/ml) using RebaudiosideA as substrate with Mn2+ as metal ion.**

| uM substrate | Steviol | Stevioside | RebaudiosideB | RebaudiosideA | RebaudiosideD |
|---|---|---|---|---|---|
| 0 | 0 | 0 | 5 | 34 | 0,6 |
| 30 | 0 | 0 | 10 | 31 | 0 |
| 300 | 0 | 0,4 | 42 | 172 | 0 |
| 3000 | 0 | 0 | 0 | 1855 | 0 |

**Table 25. Effect of metal ions on Rebaudioside glycosylation. Steviol glycosides (in ug/ml) using RebaudiosideA as substrate at 300 uM**

| Metal | Steviol | Stevioside | RebaudiosideB | RebaudiosideA | RebaudiosideD |
|---|---|---|---|---|---|
| Mn2+ | 0 | 0,4 | 42 | 172 | 0 |
| Mg2+ | 0 | 0,3 | 150 | 106 | 0,5 |
| None | 0 | 0,7 | 141 | 125 | 0,8 |

### Example 19. Diterpenes or glycosylated diterpenes production by Saccharomyces cerevisiae (comparative example)

### 19.1 Construction of recombinant host for diterpenes or glycosylated diterpenes production

The construction of the recombinant host for the construction of diterpenes or glycosylated diterpenes, STV006, is described above in Example 6.

### 19.2 Detection of rebaudioside A

At different fermentation times, aliquots of the cultures were removed. Fermentation broth was treated with heat and acetonitrile to extract the RebA from the cells.

The Rebaudioside A (C44H70O23, Mmonoisotopic = 966.43079) concentration was measured in the by LC/MS as described below. For this purpose an UHPLC system was coupled to a triple quadrupole mass spectrometer and rebaudiosisde A was detected in MS/MS mode by the loss of a hexose unit from the protonated molecule.

A Waters acquity UPLC BEH amide column (1.7 um, 2.1*150 mm) was used with isocratic elution. The mobile phase consists of acetonitrile/10 mM NH4Ac in MilliQ water (pH 6.8), (8:2 v/v), at a flowrate of 300 ul/min. The injection volume was 5 µL, and the column temperature is kept at 30 degrees Celcius.

### 19.3 Diterpene or glycosylated diterpene fermentation

The yeast strain STV006 constructed as described above, was cultivated in shake-flask (500ml with 50 ml medium) for 2 days at 30°C and 280 rpm. The medium was based on Verduyn et al. (Verduyn C, Postma E, Scheffers WA, Van Dijken JP. Yeast, 1992 Jul;8(7):501-517), with modifications in the carbon and nitrogen sources, as described in Table 26.

**Table 26. Preculture medium composition**

| **Raw material** | **Formula** | **Concentration (g/kg)** |
|---|---|---|
| Galactose | C₆H₁₂O₆ | 20.0 |
| Urea | (NH₂)₂CO | 2.3 |
| Potassium dihydrogen phosphate | KH₂PO₄ | 3.0 |
| Magnesium sulphate | MgSO₄ . 7H₂O | 0.5 |
| Trace element solution | | 1 |
| Vitamin solution | | 1 |

**^{a}Trace elements solution**

| **Component** | **Formula** | **Concentration (g/kg)** |
|---|---|---|
| EDTA | C₁₀H₁₄N₂Na₂O₈ . 2H₂O | 15.00 |
| Zincsulphate . 7H₂O | ZnSO₄.7H₂O | 4.50 |
| Manganesechloride . 2H₂O | MnCl₂ . 2H₂O | 0.84 |
| Cobalt (II) chloride. 6H₂O | CoCl₂ . 6H₂O | 0.30 |
| Cupper (II) sulphate . 5H₂O | CuSO₄ . 5H₂O | 0.30 |
| Sodium molybdenum . 2H₂O | Na₂MoO₄ . 2H₂O | 0.40 |
| Calciumchloride . 2H₂O | CaCl₂. 2H₂O | 4.50 |
| Ironsulphate . 7H₂O | FeSO₄.7H₂O | 3.00 |
| Boric acid | H₃BO₃ | 1.00 |
| Potassium iodide | KI | 0.10 |

**^{b}Vitamin solution**

| **Component** | **Formula** | **Concentration (g/kg)** |
|---|---|---|
| Biotin (D-) | C₁₀H₁₆N₂O₃S | 0.05 |
| Ca D(+) panthothenate | C₁₈H₃₂CaN₂O₁₀ | 1.00 |
| Nicotinic acid | C₆H₅NO₂ | 1.00 |
| Myo-inositol | C₆H₁₂O₆ | 25.00 |
| Thiamine chloride hydrochloride | C₁₂H₁₈Cl₂N₄OS . xH₂O | 1.00 |
| Pyridoxol hydrochloride | C₈H₁₂ClNO₃ | 1.00 |
| p-aminobenzoic acid | C₇H₇NO₂ | 0.20 |

Subsequently, 6ml of the content of the shake-flask was transferred into a fermenter (starting volume 0.3 L), which contained the medium as set out in Table 27.

**Table 27. Composition fermentation medium**

| **Raw material** | | **Final Concentration (g/kg)** |
|---|---|---|
| Ammonium sulphate | (NH₄)₂SO₄ | 1 |
| Potassium dihydrogen phosphate | KH₂PO₄ | 10 |
| Magnesium sulphate | MgSO₄ . 7H₂O | 5 |
| Trace element solution | - | 8 |
| Vitamin solution | - | 8 |

The pH was controlled at 5.0 by addition of ammonia (12.5 wt%). Temperature was controlled at 27°C, 30°C or 33°C. pO₂ was controlled at 40% by adjusting the stirrer speed. Glucose concentration was kept limited by controlled feed to the fermenter.

**Table 28. Composition of the fermentation feed medium**

| **Raw material** | **Formula** | **Final Concentration (g/kg)** |
|---|---|---|
| Glucose.1aq | C₆H₁₂O₆.1 aq | 330 |
| Potassium dihydrogen phosphate | KH₂PO₄ | 10 |
| Magnesium sulphate heptahydrate | MgSO₄.7H₂O | 5 |
| Verduyn trace elements solution | | 8 |
| Verduyn vitamin solution | | 8 |

**Table 29. RebA production (mg/L) in the fermentation broth during fed-batch fermentation at 27°C, 30°C and 33°C**

| | **Temperature** | | |
|---|---|---|---|
| **Time (h)** | **27°C** | **30°C** | **33°C** |
| 24 | 0 | 0 | 0 |
| 48 | 9 | 5 | 0 |
| 72 | 113 | 90 | 39 |
| 96 | 196 | 122 | 54 |
| 120 | 259 | 147 | 62 |
| 144 | 300 | 157 | 64 |

The results set out in Table 4 show that fermentation of a recombinant *Saccharomyces cerevisiae* at 27°C results in higher production of RebA as compared with fermentation at the normal temperature of 30°C.

**Table 1: Description of the sequence listing**

| **Nucleic acid (CpO for *S*. *cerevisiae*)** | **Nucleic acid (CpO for *Y. lipolytica*)** | **Amino acid** | **Id^{*}** | **UniProt^{^}** | **Organism** |
|---|---|---|---|---|---|
| SEQ ID NO: 1 | SEQ ID NO: 151 | SEQ ID NO: 2 | CPS_1 | Q9FXV9 | Lactuca sativa (Garden Lettuce) |
| SEQ ID NO: 3 | SEQ ID NO: 152 | SEQ ID NO: 4 | tCPS_1 | Q9FXV9 | Lactuca sativa (Garden Lettuce) |
| SEQ ID NO: 5 | SEQ ID NO: 153 | SEQ ID NO: 6 | CPS_2 | D2X8G0 | Picea glauca |
| SEQ ID NO: 7 | SEQ ID NO: 154 | SEQ ID NO: 8 | CPS_3 | Q45221 | Bradyrhizobium japonicum |
| SEQ ID NO: 9 | SEQ ID NO: 155 | SEQ ID NO: 10 | KS_1 | Q9FXV8 | Lactuca sativa (Garden Lettuce) |
| SEQ ID NO: 11 | SEQ ID NO: 156 | SEQ ID NO: 12 | tKS_1 | Q9FXV8 | Lactuca sativa (Garden Lettuce) |
| SEQ ID NO: 13 | SEQ ID NO: 157 | SEQ ID NO: 14 | KS_2 | D2X8G1 | Picea glauca |
| SEQ ID NO: 15 | SEQ ID NO: 158 | SEQ ID NO: 16 | KS_3 | Q45222 | Bradyrhizobium japonicum |
| SEQ ID NO: 17 | SEQ ID NO: 159 | SEQ ID NO: 18 | CPSKS_1 | O013284 | Phaeosphaeria sp |
| SEQ ID NO: 19 | SEQ ID NO: 160 | SEQ ID NO: 20 | CPSKS_2 | Q9UVY5 | Gibberella fujikuroi |
| SEQ ID NO: 21 | SEQ ID NO: 161 | SEQ ID NO:22 | KO_1 | B5MEX5 | Lactuca sativa (Garden Lettuce) |
| SEQ ID NO: 23 | SEQ ID NO: 162 | SEQID NO:24 | KO_2 | B5MEX6 | Lactuca sativa (Garden Lettuce) |
| SEQ ID NO: 25 | SEQ ID NO: 163 | SEQ ID NO: 26 | KO_3 | B5DBY4 | Sphaceloma manihoticola |
| SEQ ID NO: 27 | SEQ ID NO: 164 | SEQ ID NO:28 | KAH_1 | Q2HYU7 | Artemisia annua (Sweet wormwood). |
| SEQ ID NO: 29 | SEQ ID NO: 165 | SEQ ID NO:30 | KAH_2 | B9SBP0 | Ricinus communis (Castor bean). |
| SEQ ID NO: 31 | SEQ ID NO: 166 | SEQ ID NO:32 | **KAH_3** | Q0NZP1 | Stevia rebaudiana |
| SEQ ID NO: 33 | SEQ ID NO: 167 | SEQ ID NO:34 | **KAH_4** | JP20090658 86 | Arabidopsis thaliana (Mouse-ear cress) |
| SEQ ID NO: 35 | SEQ ID NO: 168 | SEQID NO:36 | UGT1_1 | A9X3L6 | Ixeris dentata var. albiflora. |
| SEQ ID NO: 37 | SEQ ID NO: 169 | SEQ ID NO:38 | UGT1_2 | B9SIN2 | Ricinus communis (Castor bean). |
| SEQ ID NO: 39 | SEQ ID NO: 170 | SEQ ID NO:40 | UGT3_1 | A9X3L7 | Ixeris dentata var. Albiflora |
| SEQ ID NO: 41 | SEQ ID NO: 171 | SEQ ID NO:42 | UGT3_2 | B9IEM5 | Populus trichocarpa (Western balsam poplar) |
| SEQ ID NO: 43 | SEQ ID NO: 172 | SEQ ID NO:44 | UGT3_3 | Q9M6E7 | Nicotiana tabacum |
| SEQ ID NO: 45 | SEQ ID NO: 173 | SEQ ID NO:46 | UGT3_4 | A3E7Y9 | Vaccaria hispanica |
| SEQ ID NO: 47 | SEQ ID NO: 174 | SEQ ID NO: 48 | UGT3_5 | P10249 | Streptococcus mutans |
| SEQ ID NO: 49 | SEQ ID NO: 175 | SEQ ID NO:50 | UGT4_1 | A4F1T4 | Lobelia erinus (Edging lobelia) |
| SEQ ID NO: 51 | SEQ ID NO: 176 | SEQ ID NO:52 | UGT4_2 | Q9M052 | Arabidopsis thaliana (Mouse-ear cress) |
| SEQ ID NO: 53 | SEQ ID NO: 177 | SEQ ID NO:54 | CPR_1 | Q7Z8R1 | Gibberella fujikuroi |
| SEQ ID NO: 55 | SEQ ID NO: 178 | SEQ ID NO:56 | CPR_2 | Q9SB48 | Arabidopsis thaliana (Mouse-ear cress) |
| SEQ ID NO: 57 | SEQ ID NO: 179 | SEQ ID NO:58 | CPR_3 | Q9SUM3 | Arabidopsis thaliana (Mouse-ear cress) |
| SEQ ID NO: 59 | SEQ ID NO: 141 | SEQ ID NO:60 | CPS_SR | O22667 | Stevia rebaudiana |
| SEQ ID NO: 61 | SEQ ID NO: 142 | SEQ ID NO: 62 | tCPS_SR | | Stevia rebaudiana |
| SEQ ID NO: 63 | SEQ ID NO: 143 | SEQ ID NO:64 | KS_SR | Q9XEI0 | Stevia rebaudiana |
| SEQ ID NO: 65 | SEQ ID NO: 144 | SEQ ID NO: 66 | tKS_SR | | Stevia rebaudiana |
| SEQ ID NO: 67 | SEQ ID NO: 145 | SEQ ID NO: 68 | KO_SR | Q4VCL5 | Stevia rebaudiana |
| SEQ ID NO: 69 | SEQ ID NO: 146 | SEQ ID NO: 70 | **KAH_SR** | US7927851 | Stevia rebaudiana |
| SEQ ID NO: 71 | SEQ ID NO: 147 | SEQ ID NO:72 | UGT1_SR | Q6VAB0 | Stevia rebaudiana |
| SEQ ID NO: 73 | SEQ ID NO: 148 | SEQ ID NO: 74 | UGT3_SR | Q6VAA6 | Stevia rebaudiana |
| SEQ ID NO: 75 | SEQ ID NO: 149 | SEQ ID NO: 76 | UGT4_SR | Q6VAB4 | Stevia rebaudiana |
| SEQ ID NO: 77 | SEQ ID NO: 150 | SEQ ID NO: 78 | CPR_SR | Q2I6J8 | Stevia rebaudiana |
| SEQ ID NO: 79 | | SEQ ID NO: 80 | tHMG1 | G2WJY0 | Saccharomyces cerevisiae |
| SEQ ID NO: 81 | | SEQID NO:82 | ERG20 | E7LW73 | Saccharomyces cerevisiae |
| SEQ ID NO: 83 | | SEQ ID NO: 84 | BTS1 | E7Q9V5 | Saccharomyces cerevisiae |
| SEQ ID NO: 85 | SEQ ID NO: 180 | SEQ ID NO: 86 | KO_Gibfu | O94142 | Gibberella fujikuroi |
| SEQ ID NO: 87 | SEQ ID NO: 181 | SEQID NO:88 | UGT2_1a | B3VI56/99% | Stevia rebaudiana |
| SEQ iD NO: 89 | | SEQ ID NO:90 | KAH_ASR1 | Xxx | S. rebaudiana |
| SEQ ID NO: 91 | | SEQ ID NO:92 | KAH_ASR2 | Q0NZP1_STE RE | S. rebaudiana |
| SEQ ID NO: 93 | | SEQ ID NO: 94 | KAH_AAT | Q6NKZ8_AR ATH | A. thaliana |
| SEQ ID NO: 95 | | SEQ ID NO:96 | KAH_AVV | F6H1G0_VIT VI/98% | Vitis vinifera |
| SEQ ID NO: 97 | | SEQ ID NO: 98 | KAH_AMT | Q2MJ20_ME DTR | Medicago truncatula |
| SEQ ID NO: 99 | | SEQ ID NO: 100 | UGT2_1b | B3VI56/99% | S. rebaudiana |
| SEQ ID NO: 101 | | SEQ ID NO: 102 | UGT2_2 | Q53UH5_IPO PU | I. purpurea |
| SEQ ID NO: 103 | | SEQ ID NO: 104 | UGT2_3 | UGAT_BELPE /99% | Bellis perennis |
| SEQ ID NO: 105 | | SEQ ID NO: 106 | UGT2_4 | B3VI56 | S. rebaudiana |
| SEQ iD NO: 107 | | SEQ ID NO: 108 | UGT2_5 | Q6VAA8 | S. rebaudiana |
| SEQ ID NO: 109 | | SEQ ID NO: 110 | UGT2_6 | Q8LKG3 | S. rebaudiana |
| SEQ ID NO: 111 | | SEQ ID NO: 112 | UGT2_7 | B9HSH7_PO PTR | Populus trichocarpa |
| SEQ ID NO: 113 | | SEQ ID NO: 114 | UGT_RD1 | Q6VAA3 | S. rebaudiana |
| SEQ ID NO: 115 | | SEQ ID NO: 116 | UGT_RD2 | Q8H6A4 | S. rebaudiana |
| SEQ ID NO: 117 | | SEQ ID NO: 118 | UGT_RD3 | Q6VAA4 | S. rebaudiana |
| SEQ ID NO: 119 | | SEQ ID NO: 120 | UGT_RD4 | Q6VAA5 | S. rebaudiana |
| SEQ ID NO: 121 | | SEQ ID NO: 122 | UGT_RD5 | Q6VAA7 | S. rebaudiana |
| SEQ ID NO: 123 | | SEQ ID NO: 124 | UGT_RD6 | Q6VAA8 | S. rebaudiana |
| SEQ ID NO: 125 | | SEQ ID NO: 126 | UGT_RD7 | Q6VAA9 | S. rebaudiana |
| SEQ ID NO: 127 | | SEQ ID NO: 128 | UGT_RD8 | Q6VAB1 | S. rebaudiana |
| SEQ ID NO: 129 | | SEQ ID NO: 130 | UGT_RD9 | Q6VAB2 | S. rebaudiana |
| SEQ ID NO: 131 | | SEQ ID NO: 132 | UGT_RD10 | Q6VAB3 | S. rebaudiana |
| SEQ ID NO: 133 | | SEQ ID NO: 134 | UGT_RD11 | B9VVB1 | S. rebaudiana |
| SEQ ID NO: 135 | | SEQ ID NO: 136 | UGT_RD12 | C7EA09 | S. rebaudiana |
| SEQ ID NO: 137 | | SEQ ID NO: 138 | UGT_RD13 | Q8LKG3 | S. rebaudiana |
| SEQ ID NO: 139 | | SEQ ID NO: 140 | UGT_RD14 | B3VI56 | S. rebaudiana |
| | SEQ ID NO: 182 | | tCPS | | |
| | SEQ ID NO: 183 | | tKS | | |
| | SEQ ID NO: 184 | | CPSKS | | |
| | SEQ ID NO: 185 | | KAH4 | | |
| | SEQ ID NO: 186 | | KO_Gibfu | | |
| | SEQ ID NO: 187 | | CPR1 | | |
| | SEQ ID NO: 188 | | CPR3 | | |
| | SEQ ID NO: 189 | | UGT1 | | |
| | SEQ ID NO: 190 | | UGT3 | | |
| | SEQ ID NO: 191 | | UGT4 | | |
| | SEQ ID NO: 192 | | UGT2_1a | | |
| | SEQ ID NO: 193 | | pTPI | | |
| | SEQ ID NO: 194 | | gpdT-pGPD | | |
| | SEQ ID NO: 195 | | pgmT-pTEF | | |
| | SEQ ID NO: 196 | | pgkT-pPGM | | |
| | SEQ ID NO: 197 | | LEU2 and flanking sequences | | |
| | SEQ ID NO: 198 | | vector sequences | | |
| | SEQ ID NO: 199 | | pENO | | |
| | SEQ ID NO: 200 | | HPH | | |
| SEQ ID NO: 201 | | | Sc Eno2.pro | | |
| SEQ ID NO: 202 | | | Sc Fba1.pro | | |
| SEQ ID NO: 203 | | | Sc Tef1.pro | | |
| SEQ ID NO: 204 | | | Sc Pgk1.pro | | |
| SEQ ID NO: 205 | | | Kl prom 12.pro | | |
| SEQ ID NO: 206 | | | Ag lox_TEF1.pro | | |
| SEQ ID NO: 207 | | | Kl prom 6.pro | | |
| SEQ ID NO: 208 | | | Sc Pma1.pro | | |
| SEQ ID NO: 209 | | | Sc Vps68.pro | | |
| SEQ ID NO: 210 | | | Sc Oye2.pro | | |
| SEQ ID NO: 211 | | | KANMX ORF | | |
| SEQ ID NO: 212 | | | Adh1.ter | | |
| SEQ ID NO: 213 | | | Adh2.ter | | |
| SEQ ID NO: 214 | | | Gmp1.ter | | |
| SEQ ID NO: 215 | | | Sc Tal1.ter | | |
| SEQ ID NO: 216 | | | Sc Tpi1.ter | | |
| SEQ ID NO: 217 | | | Ag Tef1_lox.ter | | |
| SEQ ID NO: 218 | | | Sc Pdc1.ter | | |
| SEQ ID NO: 219 | | | Sc Tdh1.ter | | |
| SEQ ID NO: 220 | | | Sc Eno1.ter | | |
| SEQ ID NO: 221 | | | Kl prom3.pro | | |
| SEQ ID NO: 222 | | | Kl prom2.pro | | |
| SEQ ID NO: 223 | | | Sc PRE3. Pro | | |

| | | | | | |
|---|---|---|---|---|---|
| greyed out ids are truncated and thus a fragment of mentioned UniProt id | | | | | |

## Claims

1. A recombinant microorganism which is a *Yarrowia,* such as *Yarrowia lipolytica,* comprising one or more nucleotide sequence(s) encoding:
a polypeptide having ent-copalyl pyrophosphate synthase activity;
a polypeptide having ent-Kaurene synthase activity;
a polypeptide having ent-Kaurene oxidase activity;
a polypeptide having kaurenoic acid 13-hydroxylase activity,
a polypeptide having NADPH-cytochrome p450 reductase activity; and
a polypeptide having UDP-glucosyltransferase activity,
wherein a nucleotide sequence encoding a polypeptide having ent-Kaurene oxidase activity encodes a polypeptide comprising an amino acid sequence that has at least 20% sequence identity with the amino acid sequence of SEQ ID NO: 86; and
wherein a nucleotide sequence encoding a polypeptide having kaurenoic acid 13-hydroxylase activity encodes a polypeptide comprising an amino acid sequence that has at least 20% sequence identity with the amino acid sequence of SEQ ID NO: 34,
whereby expression of the nucleotide sequence(s) confer(s) on the microorganism the ability to produce at least one of steviolmonoside, steviolbioside, stevioside or rebaudioside A, rebaudioside B, rebaudioside C, rebaudioside D, rebaudioside E, rebaudioside F, rubusoside, dulcoside A.

2. A recombinant microorganism according to claim 1, wherein the microorganism comprises a nucleotide sequence encoding a polypeptide capable of catalyzing the addition of a C-13-glucose to steviol,
whereby expression of the nucleotide sequence confers on the microorganism the ability to produce at least steviolmonoside.

3. A recombinant microorganism according to claim 1 or 2, wherein the microorganism comprises a nucleotide sequence encoding a polypeptide capable of catalyzing the addition of a glucose at C-13 position of steviol or steviolmonoside,
whereby expression of the nucleotide sequence confers on the microorganism the ability to produce at least steviolbioside.

4. A recombinant microorganism according to any one of claims 1 to 3, wherein the microorganism comprises a nucleotide sequence encoding a polypeptide capable of catalyzing the addition of a C-19-glucose to steviolbioside,
whereby expression of the nucleotide sequence confers on the microorganism the ability to produce at least stevioside.

5. A recombinant microorganism according to any one of claims 1 to 4, wherein the microorganism comprises a nucleotide sequence encoding a polypeptide capable of catalyzing glucosylation of the C-3' of the glucose at the C-13 position of stevioside,
whereby expression of the nucleotide sequence confers on the microorganism the ability to produce at least rebaudioside A.

6. A recombinant microorganism according to any one of claims 1 to 5, wherein the microorganism comprises a nucleotide sequence encoding a polypeptide capable of catalyzing the glucosylation of stevioside or rebaudioside A,
whereby expression of the nucleotide sequence confers on the microorganism the ability to produce at least rebaudioside D.

7. A recombinant microorganism according to any one of claims 1 to 6, wherein the microorganism comprises a nucleotide sequence encoding a polypeptide capable of catalyzing the glucosylation of stevioside,
whereby expression of the nucleotide sequence confers on the microorganism the ability to produce at least rebaudioside E.

8. A recombinant microorganism according to any one of claims 1 to 7, wherein the microorganism comprises a nucleotide sequence encoding a polypeptide capable of catalyzing the glucosylation of rebaudioside E,
whereby expression of the nucleotide sequence confers on the microorganism the ability to produce at least rebaudioside D.

9. A recombinant microorganism according to any one of the preceding claims, wherein the ability of the microorganism to produce geranylgeranyl diphosphate (GGPP) is upregulated.

10. A recombinant microorganism according to claim 9, comprising one or more nucleotide sequence(s) encoding hydroxymethylglutaryl-CoA reductase, farnesyl-pyrophosphate synthetase and geranylgeranyl diphosphate synthase, whereby expression of the nucleotide sequence(s) confer(s) on the microorganism the ability to produce elevated levels of GGPP.

11. A process for the preparation of a diterpene or glycosylated diterpene which comprises fermenting a microorganism according to any one of the preceding claims in a suitable fermentation medium, optionally at a temperature of about 29°C or less and optionally recovering the diterpene or glycosylated diterpene.

12. A method for converting a first glycosylated diterpene into a second glycosylated diterpene, which method comprises:
contacting said first glycosylated diterpene with a microorganism according to any one of claims 1 to 10,
thereby to convert the first glycosylated diterpene into the second glycosylated diterpene.

## Patentansprüche

1. Rekombinanter Mikroorganismus, bei dem es sich um eine *Yarrowia,* wie *Yarrowia lipolytica,* handelt, umfassend eine oder mehrere Nukleotidsequenz(en), die für Folgendes codiert/codieren:
ein Polypeptid mit ent-Copalylpyrophosphatsynthaseaktivität;
ein Polypeptid mit ent-Kaurensynthaseaktivität;
ein Polypeptid mit ent-Kaurenoxydaseaktivität;
ein Polypeptid mit Kaurensäure-13-Hydroxylase-aktivität;
ein Polypeptid mit NADPH-Cytochrom-p450-Reduktase-aktivität; und
ein Polypeptid mit UDP-Glycosyltransferaseaktivität,
wobei eine Nukleotidsequenz, die für ein Polypeptid mit ent-Kaurenoxidaseaktivität codiert, für ein Polypeptid umfassend eine Aminosäuresequenz mit mindestens 20 % Sequenzidentität zu der Aminosäuresequenz gemäß SEQ ID NO: 86 codiert; und
wobei eine Nukleotidsequenz, die für ein Polypeptid mit Kaurensäure-13-hydrxylaseaktivität codiert, für ein Polypeptid umfassend eine Aminosäuresequenz mit mindestens 20 % Sequenzidentität zu der Aminosäuresequenz gemäß SEQ ID NO: 34 codiert;
wobei die Expression der Nukleotidsequenz(en) dem Mikroorganismus die Fähigkeit, mindestens eines aus der Reihe Steviolmonosid, Steviolbiosid, Steviosid oder Rebaudiosid A, Rebaudiosid B, Rebaudiosid C, Rebaudiosid D, Rebaudiosid E, Rebaudiosid F, Rubusosid, Dulcosid A zu produzieren, verleiht.

2. Rekombinanter Mikroorganismus nach Anspruch 1, wobei der Mikroorganismus eine Nukleotidsequenz umfasst, die für ein Polypeptid codiert, welches fähig ist, die Addition einer C-13-Glukose an Steviol zu katalysieren,
wobei die Expression der Nukleotidsequenz dem Mikroorganismus die Fähigkeit, mindestens Steviolmonosid zu produzieren, verleiht.

3. Rekombinanter Mikroorganismus nach Anspruch 1 oder 2, wobei der Mikroorganismus eine Nukleotidsequenz umfasst, die für ein Polypeptid codiert, welches fähig ist, die Addition einer Glukose in der C-13-Position von Steviol oder Steviolmonosid zu katalysieren,
wobei die Expression der Nukleotidsequenz dem Mikroorganismus die Fähigkeit, mindestens Steviolbiosid zu produzieren, verleiht.

4. Rekombinanter Mikroorganismus nach einem der Ansprüche 1 bis 3, wobei der Mikroorganismus eine Nukleotidsequenz umfasst, die für ein Polypeptid codiert, welches fähig ist, die Addition einer C-19-Glukose an Steviolbiosid zu katalysieren,
wobei die Expression der Nukleotidsequenz dem Mikroorganismus die Fähigkeit, mindestens Steviosid zu produzieren, verleiht.

5. Rekombinanter Mikroorganismus nach einem der Ansprüche 1 bis 4, wobei der Mikroorganismus eine Nukleotidsequenz umfasst, die für ein Polypeptid codiert, welches fähig ist, die Glucosylierung des C-3' der Glucose an der C-13-Position von Steviosid zu katalysieren,
wobei die Expression der Nukleotidsequenz dem Mikroorganismus die Fähigkeit, mindestens Rebaudiosid A zu produzieren, verleiht.

6. Rekombinanter Mikroorganismus nach einem der Ansprüche 1 bis 5, wobei der Mikroorganismus eine Nukleotidsequenz umfasst, die für ein Polypeptid codiert, das fähig ist, die Glucosylierung von Steviosid oder Rebaudiosid A zu katalysieren,
wobei die Expression der Nukleotidsequenz dem Mikroorganismus die Fähigkeit, mindestens Rebaudiosid D zu produzieren, verleiht.

7. Rekombinanter Mikroorganismus nach einem der Ansprüche 1 bis 6, wobei der Mikroorganismus eine Nukleotidsequenz umfasst, die für ein Polypeptid codiert, das fähig ist, die Glucosylierung von Steviosid zu katalysieren,
wobei die Expression der Nukleotidsequenz dem Mikroorganismus die Fähigkeit, mindestens Rebaudiosid E zu produzieren, verleiht.

8. Rekombinanter Mikroorganismus nach einem der Ansprüche 1 bis 7, wobei der Mikroorganismus eine Nukleotidsequenz umfasst, die für ein Polypeptid codiert, das fähig ist, die Glucosylierung von Rebaudiosid E zu katalysieren,
wobei die Expression der Nukleotidsequenz dem Mikroorganismus die Fähigkeit, mindestens Rebaudiosid D zu produzieren, verleiht.

9. Rekombinanter Mikroorganismus nach einem der vorhergehenden Ansprüche, wobei die Fähigkeit des Mikroorganismus, Geranylgeranyldiphosphat (GGPP) zu produzieren, hinaufreguliert ist.

10. Rekombinanter Mikroorganismus nach Anspruch 9, umfassend eine oder mehrere Nukleotidsequenz(en), die für Hydroxymethylglutaryl-CoA-Reduktase, Farnesylpyrophosphatsynthetase und Geranylgeranyldiphosphatsynthase codiert/codieren, wobei die Expression der Nukleotidsequenz(en) dem Mikroorganismus die Fähigkeit, erhöhte GGPP-Mengen zu produzieren, verleiht.

11. Verfahren zur Produktion eines Diterpens oder eines glykosylierten Diterpens, bei dem man einen Mikroorganismus nach einem der vorhergehenden Ansprüche in einem geeigneten Fermentationsmedium, gegebenenfalls bei einer Temperatur von ungefähr 29 °C oder weniger, fermentiert und gegebenenfalls das Diterpen oder das glykosylierte Diterpen isoliert.

12. Verfahren zum Umwandeln eines ersten glykosylierten Diterpens in ein zweites glykosyliertes Diterpen, das Folgendes umfasst:
Inkontaktbringen des ersten glykosylierten Diterpens mit einem Mikroorganismus nach einem der Ansprüche 1 bis 10,
wodurch das erste glykosylierte Diterpen in das zweite glykosylierte Diterpen umgewandelt wird.

## Revendications

1. Microorganisme recombiné, qui est de type *Yarrowia,* tel que *Yarrowia lipolytica,* comprenant une ou plusieurs séquences nucléotidiques codant pour :
un polypeptide ayant une activité de pyrophosphate d'ent-copalyle synthase ;
un polypeptide ayant une activité d'ent-kaurène synthase ;
un polypeptide ayant une activité d'ent-kaurène oxydase ;
un polypeptide ayant une activité d'acide kaurénoïque 13-hydroxylase ;
un polypeptide ayant une activité de NADPH-cytochrome p450 réductase ; et
un polypeptide ayant une activité d'UDP-glucosyltransférase ;
où une séquence nucléotidique codant pour un polypeptide ayant une activité d'ent-kaurène oxydase code pour un polypeptide comprenant une séquence d'acides aminés ayant au moins 20% d'identité de séquence avec la séquence d'acides aminés de SEQ ID n° 86 ; et
où une séquence nucléotidique codant pour un polypeptide ayant une activité d'acide kaurénoïque 13-hydroxylase code pour un polypeptide comprenant une séquence d'acides aminés ayant au moins 20% d'identité de séquence avec la séquence d'acides aminés de SEQ ID n° 34 ;
ce par quoi l'expression de la ou des séquences nucléotidiques confère au microorganisme l'aptitude à produire au moins l'un parmi le stéviolmonoside, le stéviolbioside, le stévioside ou le rébaudioside A, le rébaudioside B, le rébaudioside C, le rébaudioside D, le rébaudioside E, le rébaudioside F, le rubusoside, le dulcoside A.

2. Microorganisme recombiné selon la revendication 1, où le microorganisme comprend une séquence nucléotidique codant pour un polypeptide capable de catalyser l'addition d'un C-13-glucose à du stéviol,
ce par quoi l'expression de la séquence nucléotidique confère au microorganisme l'aptitude à produire au moins du stéviolmonoside.

3. Microorganisme recombiné selon la revendication 1 ou 2, où le microorganisme comprend une séquence nucléotidique codant pour un polypeptide capable de catalyser l'addition d'un glucose au niveau de la position C-13 du stéviol ou du stéviolmonoside,
ce par quoi l'expression de la séquence nucléotidique confère au microorganisme l'aptitude à produire au moins du stéviolbioside.

4. Microorganisme recombiné selon l'une quelconque des revendications 1 à 3, où le microorganisme comprend une séquence nucléotidique codant pour un polypeptide capable de catalyser l'addition d'un C-19-glucose à du stéviolbioside,
ce par quoi l'expression de la séquence nucléotidique confère au microorganisme l'aptitude à produire au moins du stévioside.

5. Microorganisme recombiné selon l'une quelconque des revendications 1 à 4, où le microorganisme comprend une séquence nucléotidique codant pour un polypeptide capable de catalyser la glucosylation du C-3' du glucose au niveau de la position C-13 du stévioside,
ce par quoi l'expression de la séquence nucléotidique confère au microorganisme l'aptitude à produire au moins du rébaudioside A.

6. Microorganisme recombiné selon l'une quelconque des revendications 1 à 5, où le microorganisme comprend une séquence nucléotidique codant pour un polypeptide capable de catalyser la glucosylation de stévioside ou de rébaudioside A,
ce par quoi l'expression de la séquence nucléotidique confère au microorganisme l'aptitude à produire au moins du rébaudioside D.

7. Microorganisme recombiné selon l'une quelconque des revendications 1 à 6, où le microorganisme comprend une séquence nucléotidique codant pour un polypeptide capable de catalyser la glucosylation de stévioside,
ce par quoi l'expression de la séquence nucléotidique confère au microorganisme l'aptitude à produire au moins du rébaudioside E.

8. Microorganisme recombiné selon l'une quelconque des revendications 1 à 7, où le microorganisme comprend une séquence nucléotidique codant pour un polypeptide capable de catalyser la glucosylation de rébaudioside E,
ce par quoi l'expression de la séquence nucléotidique confère au microorganisme l'aptitude à produire au moins du rébaudioside D.

9. Microorganisme recombiné selon l'une quelconque des revendications précédentes, où l'aptitude du microorganisme à produire du diphosphate de géranylgéranyle (GGPP) est régulée à la hausse.

10. Microorganisme recombiné selon la revendication 9, comprenant une ou plusieurs séquences nucléotidiques codant pour l'hydroxyméthylglutaryl-CoA réductase, la pyrophosphate de farnésyle synthétase et la diphosphate de géranylgéranyle synthase, ce par quoi l'expression de la ou des séquences nucléotidiques confère au microorganisme l'aptitude à produire des taux élevés de GGPP.

11. Procédé de préparation d'un diterpène ou d'un diterpène glycosylé, comprenant la fermentation d'un microorganisme selon l'une quelconque des revendications précédentes dans un milieu de fermentation convenable, éventuellement à une température d'environ 29°C ou moins et éventuellement la récupération du diterpène ou du diterpène glycosylé.

12. Méthode de conversion d'un premier diterpène glycosylé en un deuxième diterpène glycosylé, laquelle méthode comprend :
la mise en contact dudit premier diterpène glycosylé avec un microorganisme selon l'une quelconque des revendications 1 à 10,
pour convertir ainsi le premier diterpène glycosylé en un deuxième diterpène glycosylé.
